# EUROPEAN PATENT APPLICATION

(11) **EP 4 394 985 A1**
(43) Date of publication of application: **03.07.2024**
(21) Application number: 22875995.7
(22) Date of filing: 21.09.2022
(51) Int. Cl.: H01M 10/0567, C07C 301/00, H01M 10/052, H01M 10/0568, H01M 10/0569

(54) **NONAQUEOUS ELECTROLYTIC SOLUTION, NONAQUEOUS ELECTROLYTE SOLUTION BATTERY, COMPOUND, AND ADDITIVE FOR NONAQUEOUS ELECTROLYTE SOLUTION**

(30) Priority: 30.09.2021 JP 2021162010
(71) Applicant: Central Glass Co., Ltd., Yamaguchi 755-0001 (JP)
(72) Inventor: IWASAKI Susumu, Tokyo 101-0054 (JP); KATAOKA Fuga, Tokyo 101-0054 (JP); MORINAKA Takayoshi, Tokyo 101-0054 (JP)
(74) Representative: Manitz Finsterwald Patent- und Rechtsanwaltspartnerschaft mbB
(86) International application number: PCT/JP2022/035216
(87) International publication number: WO 2023/054128

(57) **Abstract**

Provided are a nonaqueous electrolyte solution containing: (I) a compound represented by Formula (1) described in the specification (for example, a compound represented by the following Formula (a-1)); (II) a solute; and (III) a nonaqueous organic solvent, a nonaqueous electrolyte solution and a nonaqueous electrolyte solution battery that can improve high-temperature cycle characteristics and suppress an increase in battery resistance by using the compound represented by Formula (1) and an additive for nonaqueous electrolyte solution, and a compound and an additive for nonaqueous electrolyte solution that can be suitably used in the nonaqueous electrolyte solution described above.

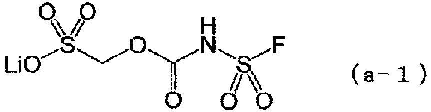

## Description

### TECHNICAL FIELD

The present disclosure relates to a nonaqueous electrolyte solution, a nonaqueous electrolyte solution battery, a compound, and an additive for nonaqueous electrolyte solution.

### BACKGROUND ART

As measures for improving cycle characteristics, high-temperature storage characteristics, and durability of a nonaqueous electrolyte solution battery, optimization of various constituting elements of the battery including active materials of a positive electrode and a negative electrode has been hitherto studied. Techniques relating to a nonaqueous electrolyte solution have also been studied, and use of a variety of additives to prevent deterioration of a nonaqueous electrolyte solution at surfaces of active positive and negative electrodes due to decomposition of the electrolyte solution has been proposed.

For example, Patent Literature 1 discloses that a negative electrode contains a hydroxyalkyl sulfonate, so that the hydroxyalkyl sulfonate shows an effect of improving cycle characteristics and reducing gas generation at a high temperature.

Patent Literature 2 discloses that a nonaqueous electrolyte solution for a lithium battery in which gas generation and a self-discharge reaction during high-temperature storage are reduced or prevented by adding a hydroxyalkyl sulfonic acid and a sulfonic acid ester in combination.

Furthermore, Patent Literature 3 discloses that adding a sulfonic acid having a urethane bond and a polymerizable unsaturated bond to an electrolyte solution allows for excellent output characteristics and long-term cycle characteristics, and an effect of decreasing electrode resistance.

### CITATION LIST

### PATENT LITERATURE

Patent Literature 1: JP5262085B
Patent Literature 2: JP5815118B
Patent Literature 3: JP6165162B

### SUMMARY OF INVENTION

### TECHNICAL PROBLEM

However, as a result of studies conducted by the present inventors, it has been found that there is room for improvement in an effect of improving cycle characteristics at a high temperature and an effect of suppressing an increase in resistance even when these sulfonic acids and sulfonate are added.

The present disclosure is made in view of the above circumstances, and an object thereof is to provide a nonaqueous electrolyte solution and a nonaqueous electrolyte solution battery that can improve high-temperature cycle characteristics and can suppress an increase in battery resistance. Another object is to provide a compound and an additive for nonaqueous electrolyte solution that can be suitably used in the above nonaqueous electrolyte solution.

### SOLUTION TO PROBLEM

In view of such problems, the present inventors conducted an extensive research and found that a nonaqueous electrolyte solution battery having improved high-temperature cycle characteristics and excellent in suppressing an increase in resistance can be obtained by using a nonaqueous electrolyte solution containing: (I) a compound represented by Formula (1) described later (hereinafter, also described as "Component (I)"); (II) a solute (hereinafter, also described as "Component (II)"); and (III) a nonaqueous organic solvent (hereinafter, also described as "Component (III)"), thereby solving the above problem.

That is, the present inventors found that the above problem can be solved by the following configurations.
[1] A nonaqueous electrolyte solution, containing: (I) a compound represented by the above Formula (1);
   (II) solute; and
   (III) a nonaqueous organic solvent. 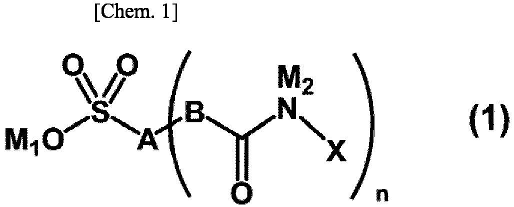
      [In Formula (1), A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms. At least one hydrogen atom in the organic group may be substituted with a fluorine atom, and an oxygen atom may be present in the organic group.
      B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms. At least one hydrogen atom in the organic group may be substituted with a halogen atom.
      M₁ and M₂ each independently represent a hydrogen atom, a metal cation, or an onium cation. When M₁ and M₂ represent a metal cation or an onium cation, a bond between the oxygen atom and M₁ and a bond between a nitrogen atom and M₂ in Formula (1) represent an ionic bond.
      X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group may be substituted with a fluorine atom, and at least one of oxygen atom or unsaturated bond may be present in the organic group. R_{c} and R_{d} may bond to each other to form a cyclic structure.
         n represents an integer of 1 to 4.]
[2] The nonaqueous electrolyte solution according to [1], in which X in Formula (1) represents -S(=O)₂-R_{b}, and the R_{b} represents a fluorine atom.
[3] The nonaqueous electrolyte solution according to [1] or [2], in which A in Formula (1) represents a linear alkylene group having 1 to 4 carbon atoms or a branched alkylene group having 2 to 4 carbon atoms.
[4] The nonaqueous electrolyte solution according to any one of [1] to [3], in which the solute is at least one selected from the group consisting of LiPF₆, LiBF₄, LiSbF₆, LiAsF₆, LiClO₄, LiCF₃SO₃, LiC₄F₉SO₃, LiN(SOzF)z, LiAlO₂, LiAlCla, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.
[5] The nonaqueous electrolyte solution according to any one of [1] to [4], in which the nonaqueous organic solvent is at least one selected from the group consisting of a cyclic ester, a chain ester, a cyclic ether, a chain ether, a sulfone compound, a sulfoxide compound, and an ionic liquid.
[6] The nonaqueous electrolyte solution according to [5], in which the nonaqueous organic solvent contains a cyclic ester, and the cyclic ester is cyclic carbonate.
[7] The nonaqueous electrolyte solution according to [6], in which the cyclic carbonate is at least one selected from the group consisting of ethylene carbonate, propylene carbonate, and fluoroethylene carbonate.
[8] The nonaqueous electrolyte solution according to [5], in which the nonaqueous organic solvent contains a chain ester, and the chain ester is chain carbonate.
[9] The nonaqueous electrolyte solution according to [8], in which the chain carbonate is at least one selected from the group consisting of ethyl methyl carbonate, dimethyl carbonate, diethyl carbonate, and methyl propyl carbonate.
[10] The nonaqueous electrolyte solution according to any one of [1] to [9], in which a content of the (I) is 0.01% by mass to 5.0% by mass with respect to a total amount of the nonaqueous electrolyte solution.
[11] The nonaqueous electrolyte solution according to any one of [1] to [10], further containing: at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.
[12] A nonaqueous electrolyte solution battery, at least including: a positive electrode; a negative electrode; a separator; and the nonaqueous electrolyte solution according to any one of [1] to [11].
[13] A compound represented by the following Formula (1). 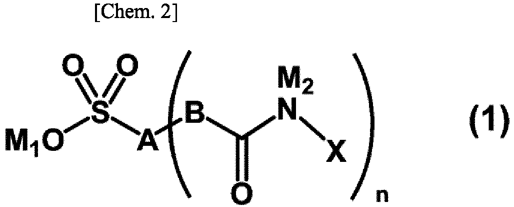 '
   [In Formula (1), A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms. At least one hydrogen atom in the organic group may be substituted with a fluorine atom, and an oxygen atom may be present in the organic group.
   B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms. At least one hydrogen atom in the organic group may be substituted with a halogen atom.
   M₁ and M₂ each independently represent a hydrogen atom, a metal cation, or an onium cation. When M₁ and M₂ represent a metal cation or an onium cation, a bond between the oxygen atom and M₁ and a bond between a nitrogen atom and M₂ in Formula (1) represent an ionic bond.
   X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group may be substituted with a fluorine atom, and at least one of oxygen atom or unsaturated bond may be present in the organic group. R_{c} and R_{d} may bond to each other to form a cyclic structure.
   n represents an integer of 1 to 4.]
[14] The compound according to [13], in which X in Formula (1) represents -S(=O)₂-R_{b}, and the R_{b} represents a fluorine atom.
[15] The compound according to [13] or [14], in which A in Formula (1) represents a linear alkylene group having 1 to 4 carbon atoms or a branched alkylene group having 2 to 4 carbon atoms.
[16] An additive for nonaqueous electrolyte solution represented by the following Formula (1). 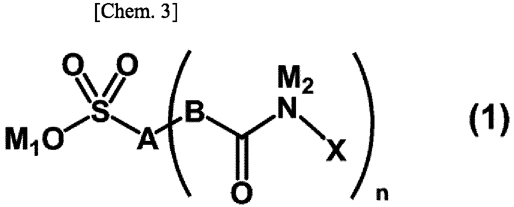
   [In Formula (1), A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms. At least one hydrogen atom in the organic group may be substituted with a fluorine atom, and an oxygen atom may be present in the organic group.
   B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms. At least one hydrogen atom in the organic group may be substituted with a halogen atom.
   M₁ and M₂ each independently represent a hydrogen atom, a metal cation, or an onium cation. When M₁ and M₂ represent a metal cation or an onium cation, a bond between the oxygen atom and M₁ and a bond between a nitrogen atom and M₂ in Formula (1) represent an ionic bond.
   X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group may be substituted with a fluorine atom, and at least one of oxygen atom or unsaturated bond may be present in the organic group. R_{c} and R_{d} may bond to each other to form a cyclic structure.
   n represents an integer of 1 to 4.]
[17] The additive for nonaqueous electrolyte solution according to [16], in which X in Formula (1) represents -S(=O)₂-R_{b}, and the R_{b} represents a fluorine atom.
[18] The additive for nonaqueous electrolyte solution according to [16] or [17], in which A in Formula (1) represents a linear alkylene group having 1 to 4 carbon atoms or a branched alkylene group having 2 to 4 carbon atoms.

### ADVANTAGEOUS EFFECTS OF INVENTION

The present disclosure allows for providing a nonaqueous electrolyte solution and a nonaqueous electrolyte solution battery that can improve high-temperature cycle characteristics and suppress an increase in battery resistance. In addition, the present disclosure also allows for providing a compound and an additive for nonaqueous electrolyte solution that can be suitably used in the above nonaqueous electrolyte solution.

### DESCRIPTION OF EMBODIMENTS

Configurations and combinations thereof in the following embodiments are merely examples, and configuration additions, replacements, and other changes are possible without departing from the scope of the present disclosure. In addition, the present disclosure is not limited by the embodiments, but only by the scope of claims.

Ranges expressed with "to" in the present specification mean ranges including numerical values indicated before and after "to" as a lower limit value and an upper limit value.

### [1. Nonaqueous Electrolyte Solution]

A nonaqueous electrolyte solution according to the present disclosure is a nonaqueous electrolyte solution containing (I) a compound represented by the above Formula (1), (II) a solute, and (III) a nonaqueous organic solvent.

### <Regarding Component (I)>

The nonaqueous electrolyte solution according to the present disclosure contains the compound represented by Formula (1), which is Component (I).

When the nonaqueous electrolyte solution containing Component (I) is used in a nonaqueous electrolyte solution battery (for example, a lithium ion secondary battery or a sodium ion secondary battery), Component (I) decomposes on at least one of a positive electrode or a negative electrode, and forms a film having good cation conductivity on a surface of at least one of the positive electrode or the negative electrode. It is considered that this film prevents direct contact between the nonaqueous organic solvent or solute and an electrode active material, and reduces cation dissociation energy of the solute. And, it is estimate by the present inventors that the film results in improvement of high-temperature cycle characteristics of the nonaqueous electrolyte solution battery and exhibits an effect of suppressing an increase in battery resistance.

The compound represented by Formula (1) is described below.

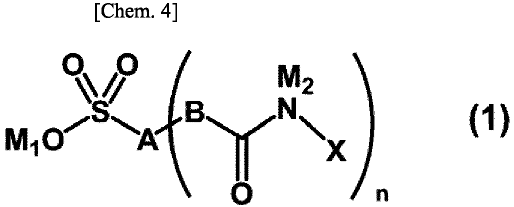

[In Formula (1), A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms. At least one hydrogen atom in the organic group may be substituted with a fluorine atom, and an oxygen atom may be present in the organic group.

B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms. At least one hydrogen atom in the organic group may be substituted with a halogen atom.

M₁ and M₂ each independently represent a hydrogen atom, a metal cation, or an onium cation. When M₁ and M₂ represent a metal cation or an onium cation, a bond between the oxygen atom and M₁ and a bond between a nitrogen atom and M₂ in Formula (1) represent an ionic bond.

X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms. At least one hydrogen atom in the organic group may be substituted with a fluorine atom, and at least one of oxygen atom or unsaturated bond may be present in the organic group. R_{c} and R_{d} may bond to each other to form a cyclic structure.
n represents an integer of 1 to 4.]

In Formula (1), A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms.

Specific examples when A represents a linear alkylene group having 1 to 8 carbon atoms or a branched alkylene group having 2 to 8 carbon atoms may include a methylene group, a methyl methylene group, an ethyl methylene group, an ethylene group, a methyl ethylene group, an ethyl ethylene group, an n-propyl ethylene group, an i-propyl ethylene group, an n-butyl ethylene group, an i-butyl ethylene group, a tert-butyl ethylene group, an n-propylene group, an i-propylene group, an n-butylene group, a 1-methyl propylene group, a 2-methyl propylene group, a tert-butylene group, an n-pentylene group, an i-pentylene group, a 1,1-dimethyl propylene group, a 1-methyl butylene group, a 1,1-dimethyl butylene group, and an n-hexylene group.

The alkylene group is preferably a linear alkylene group having 1 to 4 carbon atoms or a branched alkylene group having 2 to 4 carbon atoms.

Specific examples when A represents a linear alkenylene group having 2 to 8 carbon atoms or a branched alkenylene group having 3 to 8 carbon atoms may include a vinylene group, a propenylene group, a 1-butenylene group, a 2-butenylene group, a butadienylene group, a pentenylene group, a hexenylene group, a heptenylene group, and an octenylene group.

The alkenylene group is preferably a linear alkenylene group having 2 to 4 carbon atoms or a branched alkenylene group having 3 to 4 carbon atoms.

Any hydrogen atom of the above organic group represented by A may be substituted with a fluorine atom. Examples of the organic group in which any hydrogen atom is substituted with a fluorine atom include a -CF₂- group, a -CHF- group, a -CH₂CF₂- group, a -CH₂CHF- group, a -CH₂CH₂CHF- group, a -CH₂CH₂CF₂- group, a - CH₂CF₂CF₂- group, a -CF₂CF₂CF₂- group, a -CF=CF- group, and a -CF=CH- group.

An oxygen atom may be present in the above organic group. For example, an oxygen atom may be included between carbon atom-carbon atom bonds in the above organic group.

Specific examples when an oxygen atom is included between the carbon atom-carbon bonds in the above organic group include a -CHzCHz-O-CHz- group and a -CHzCHz-O-CHzCHz- group.

A represents preferably a linear alkylene group having 1 to 4 carbon atoms or a branched alkylene group having 2 to 4 carbon atoms, more preferably represents an unsubstituted linear alkylene group having 1 to 4 carbon atoms or an unsubstituted branched alkylene group having 2 to 4 carbon atoms, and still more preferably represents a methylene group, a methyl methylene group, an ethylene group, an n-propylene group, or an i-propylene group.

In Formula (1), B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms.

When Rₐ represents an alkyl group having 1 to 10 carbon atoms, examples of the alkyl group include a methyl group, an ethyl group, an n-propyl group, an isopropyl group, an n-butyl group, a sec-butyl group, an isobutyl group, a tert-butyl group, an n-pentyl group, an n-hexyl group, an n-octyl group, and an n-decanyl group.

When Rₐ represents an alkenyl group having 2 to 10 carbon atoms, examples of the alkenyl group include a vinyl group, an allyl group, a 1-propenyl group, an isopropenyl group, a 2-butenyl group, and a 1,3-butadienyl group.

When Rₐ represents an alkynyl group having 2 to 10 carbon atoms, examples of the alkynyl group include an ethynyl group, a 2-propynyl group, and a 1,1-dimethyl-2-propynyl group.

When Rₐ represents a cycloalkyl group having 3 to 10 carbon atoms, examples of the cycloalkyl group include a cyclopentyl group and a cyclohexyl group.

When Rₐ represents a cycloalkenyl group having 3 to 10 carbon atoms, examples of the cycloalkenyl group include a cyclopentenyl group and a cyclohexenyl group.

When Rₐ represents an aryl group having 6 to 10 carbon atoms, examples of the aryl group include a phenyl group, a tolyl group, and an xylyl group.

At least one of any hydrogen atoms of the above organic group may be substituted with a halogen atom. Examples of the halogen atom include a fluorine atom, a bromine atom, and an iodine atom, and a fluorine atom is preferable.

Rₐ preferably represents a hydrogen atom or an alkyl group having 1 to 10 carbon atoms, more preferably represents a methyl group, an ethyl group, or an isopropyl group, and further preferably represents a methyl group.

B preferably represents an oxygen atom, NH, N(CH₃), N(CH₂CH₃), or N(CH(CH₃)₂), more preferably represents an oxygen atom or N(CH₃), and further preferably represents an oxygen atom.

In Formula (1), M₁ and M₂ each independently represent a hydrogen atom, a metal cation, or an onium cation. When M₁ and M₂ represent a metal cation or an onium cation, a bond between the oxygen atom and M₁ and a bond between a nitrogen atom and M₂ in Formula (1) represent an ionic bond.

When M₁ and M₂ each represent a metal cation, examples of the metal cation include an alkali metal cation such as a lithium ion, a sodium ion, and a potassium ion.

When M₁ and M₂ each represent an onium cation, examples of the onium cation include a tetraalkylammonium ion.

M₁ preferably represents a metal cation. When the compound represented by Formula (1) is used in a lithium ion battery, a lithium ion is more preferable, and when the compound is used in a sodium ion battery, a sodium ion is more preferable.

M₂ preferably represents a hydrogen atom or a metal cation. When the compound represented by Formula (1) is used in a lithium ion battery, a lithium ion is more preferable, and when the compound is used in a sodium ion battery, a sodium ion is more preferable.

In Formula (1), X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms.

Specific examples of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, which are represented by R_{b} to R_{d}, include those examples described in the above Rₐ as an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms.

When R_{b} to R_{d} represent an alkoxy group having 1 to 10 carbon atoms, examples of an alkyl group contained in the alkoxy group include an alkyl group having 1 to 10 carbon atoms represented by R_{b} to R_{d}.

When R_{b} to R_{d} represent an alkenyloxy group having 2 to 10 carbon atoms, examples of an alkenyl group contained in the alkenyloxy group include an alkenyl group having 2 to 10 carbon atoms represented by R_{b} to R_{d}.

When R_{b} to R_{d} represent an alkynyloxy group having 2 to 10 carbon atoms, examples of an alkynyl group contained in the alkynyloxy group include an alkynyl group having 2 to 10 carbon atoms represented by R_{b} to R_{d}.

When R_{b} to R_{d} represent a cycloalkoxy group having 3 to 10 carbon atoms, examples of a cycloalkyl group contained in the cycloalkoxy group include a cycloalkyl group having 3 to 10 carbon atoms represented by R_{b} to R_{d}.

When R_{b} to R_{d} represent a cycloalkenyloxy group having 3 to 10 carbon atoms, examples of a cycloalkenyl group contained in the cycloalkenyloxy group include a cycloalkenyl group having 3 to 10 carbon atoms represented by R_{b} to R_{d}.

When R_{b} to R_{d} represent an aryloxy group having 6 to 10 carbon atoms, examples of an aryl group contained in the aryloxy group include an aryl group having 6 to 10 carbon atoms represented by R_{b} to R_{d}.

Any hydrogen atom of the above organic group represented by R_{b} to R_{d} may be substituted with a fluorine atom. Examples of the organic group in which any hydrogen atom is substituted with a fluorine atom include a trifluoromethyl group, a difluoromethyl group, a fluoromethyl group, a 2,2,2-trifluoroethyl group, a 2,2-difluoroethyl group, a 2-fluoroethyl group, a 3-fluoropropyl group, a 3,3,3-trifluoropropyl group, a 2,2,3,3,3-pentafluoropropyl group, a 2,2,3,3-tetrafluoropropyl group, a hexafluoroisopropyl group, a -CF=CF₂ group, and a - CF=CH₂ group.

An oxygen atom may be present in the above organic group. For example, an oxygen atom may be included between carbon atom-carbon atom bonds in the above organic group.

Specific examples when an oxygen atom is included between the carbon atom-carbon bonds in the above organic group include a -CH₂CH₂-O-CH₃- group and a -CH₂CH₂-O-CH₂CH₃- group.

The above R_{c} and R_{d} may bond to each other to form a cyclic structure. The cyclic structure formed by R_{c} and R_{d} together with a phosphorus atom is preferably a 5- or 6-membered ring, more preferably a 5-membered ring.

When X represents -S(=O)₂-R_{b}, R_{b} preferably represents a fluorine atom, an alkyl group having 1 to 3 carbon atoms, an alkoxy group having 1 to 3 carbon atoms, or an alkenyloxy group having 2 to 3 carbon atoms, and more preferably represents a fluorine atom, a methyl group, a trifluoromethyl group, a methoxy group, or an allyloxy group, and further preferably represents a fluorine atom.

When X represents -P(=O)-R_{c}R_{d}, R_{c} and R_{d} each independently and preferably represent a fluorine atom, an alkyl group having 1 to 3 carbon atoms, or an alkoxy group having 1 to 3 carbon atoms, more preferably represent a fluorine atom or an alkoxy group having 1 to 3 carbon atoms, and further preferably represent a fluorine atom or a methoxy group.

Preferably, R_{c} and R_{d} represent an alkoxy group having 1 or 2 carbon atoms and bond to each other to form a 5-membered ring or 6-membered ring with a phosphorus atom.

Most preferably, X represents -S(=O)₂-R_{b}, and R_{b} represents a fluorine atom.

n represents an integer of 1 to 4, preferably represents 1 to 3, and more preferably represents 1 or 2.

When n represents 1, the compound represented by Formula (1) is a compound represented by the following Formula (1-1). 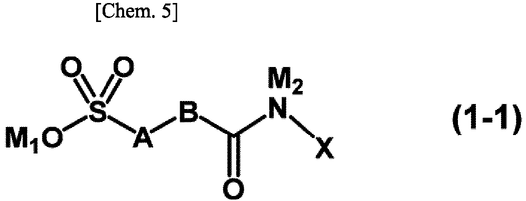 [In Formula (1-1), A, B, X, M₁, and M₂ each have the same meaning as A, B, X, M₁, and M₂ in Formula (1).]

Specific examples of the compound represented by Formula (1) are shown below, but the present invention is not limited thereto.

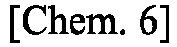

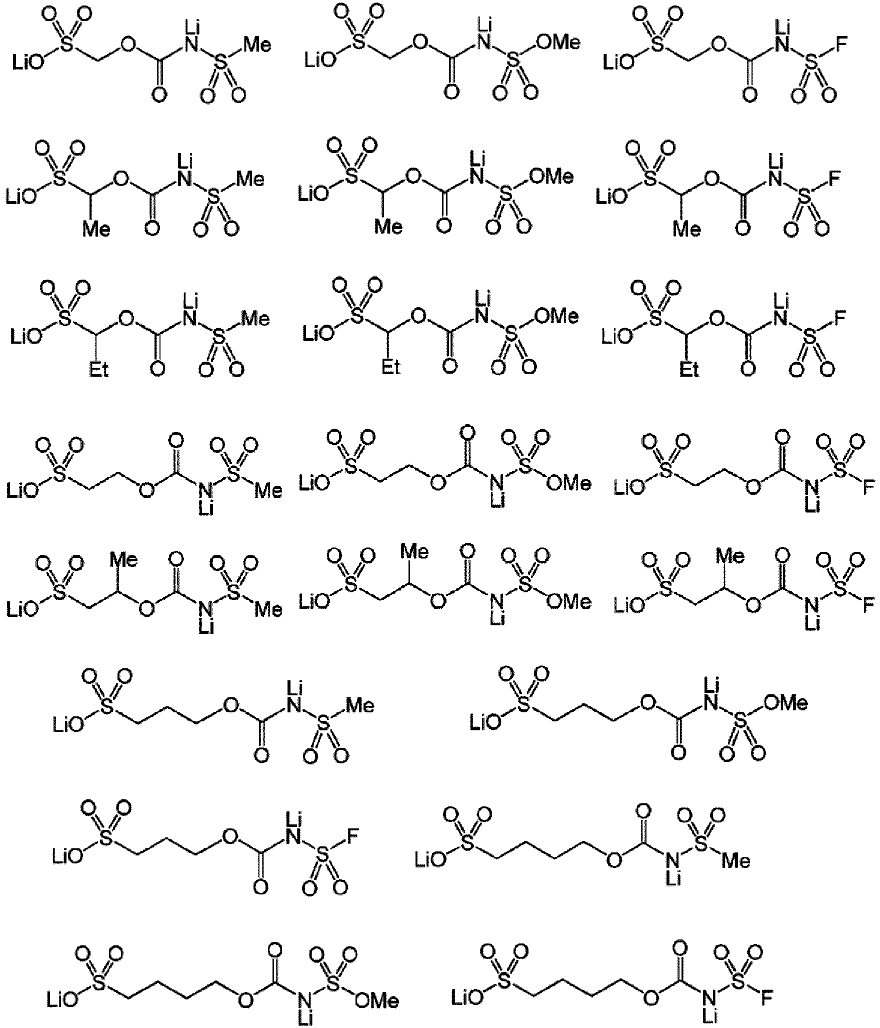

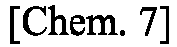

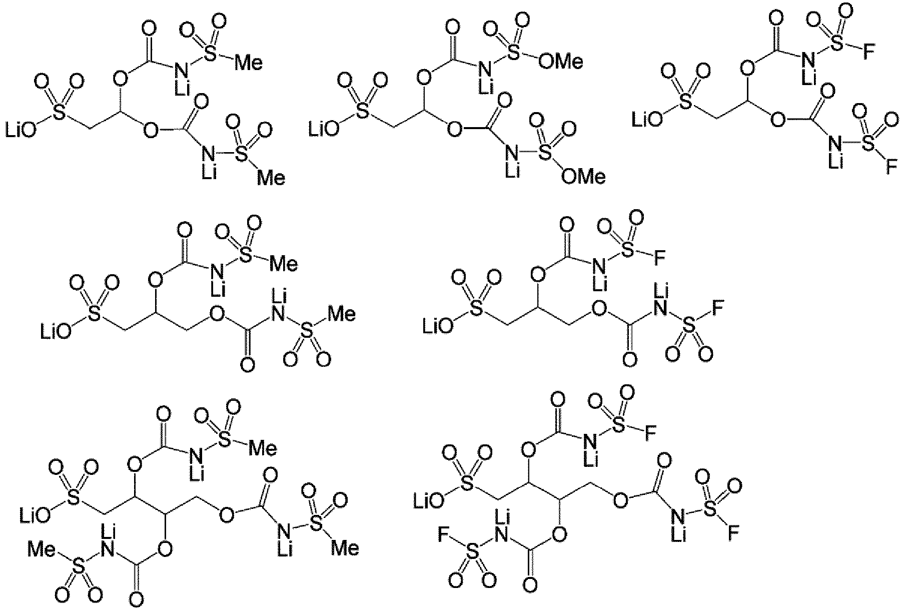

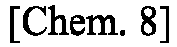

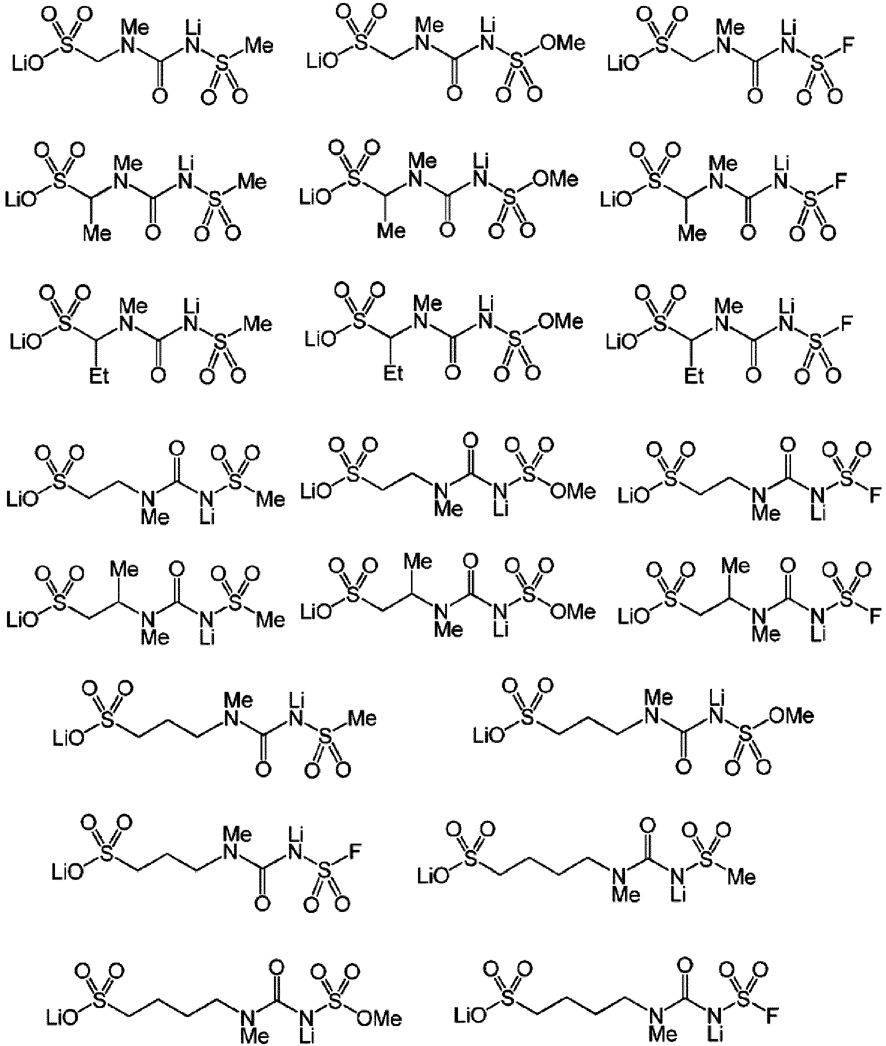

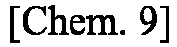

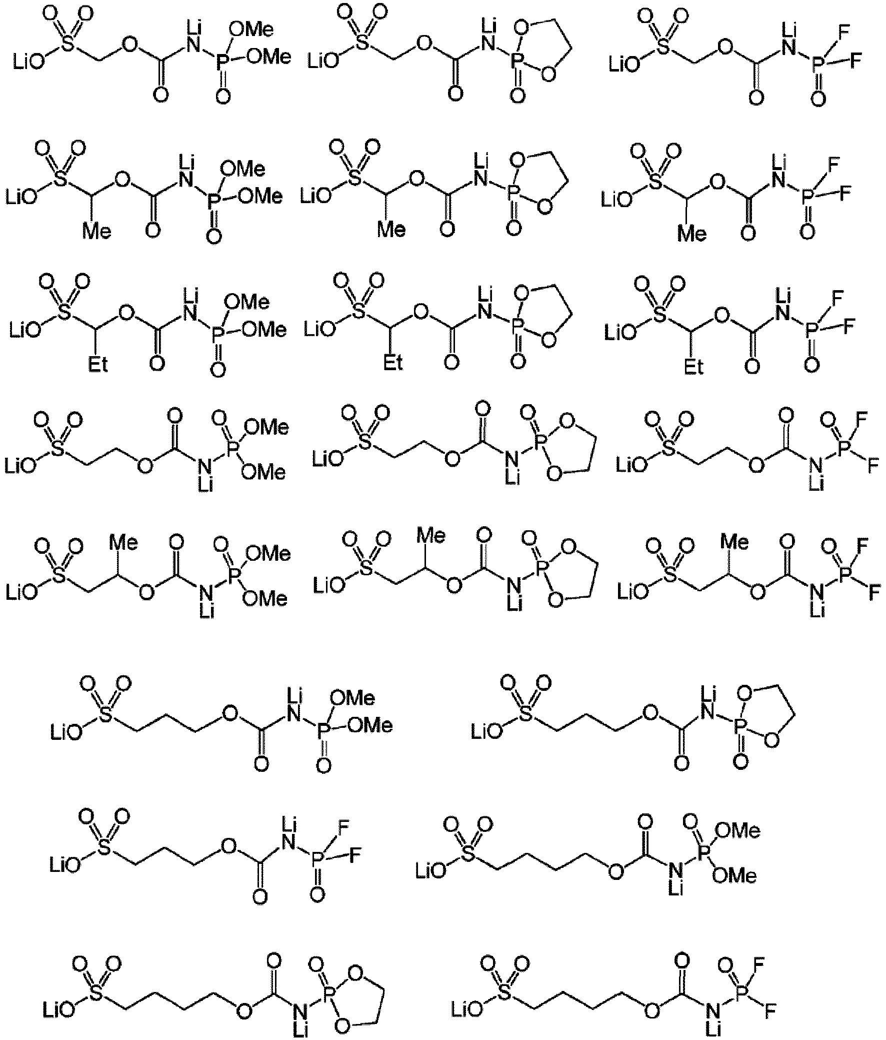

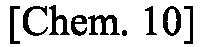

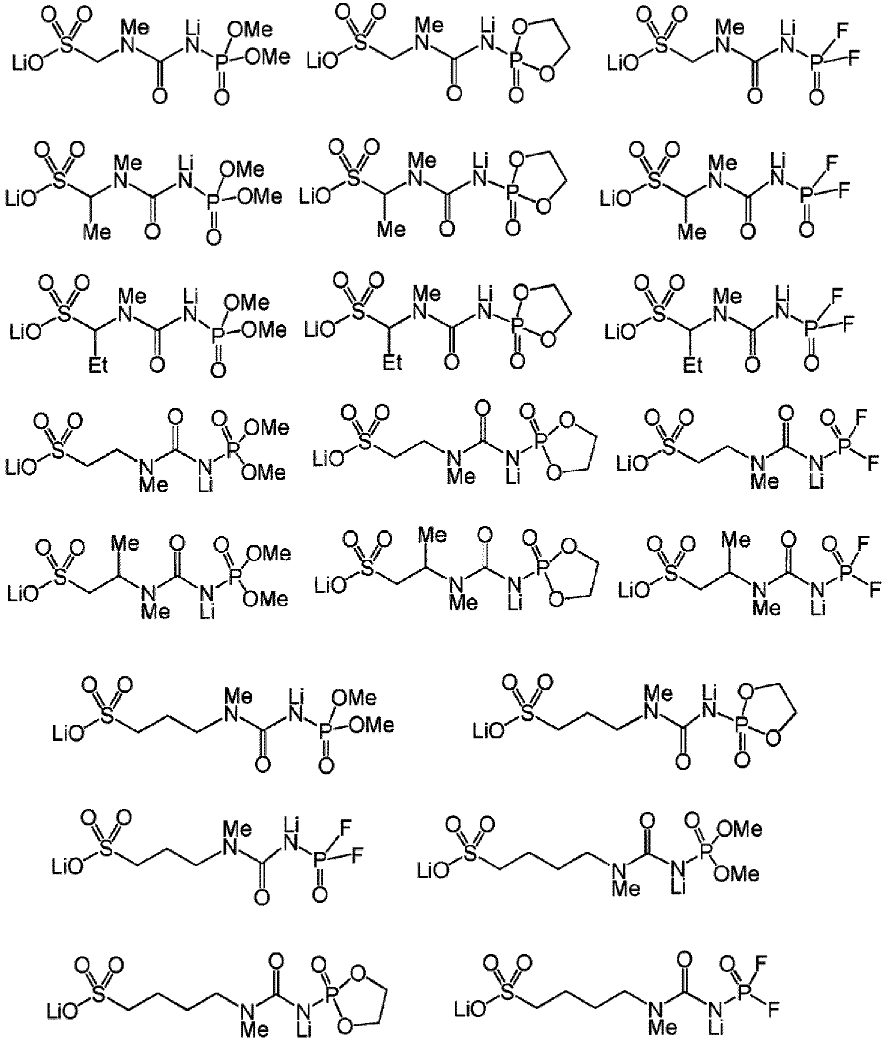

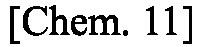

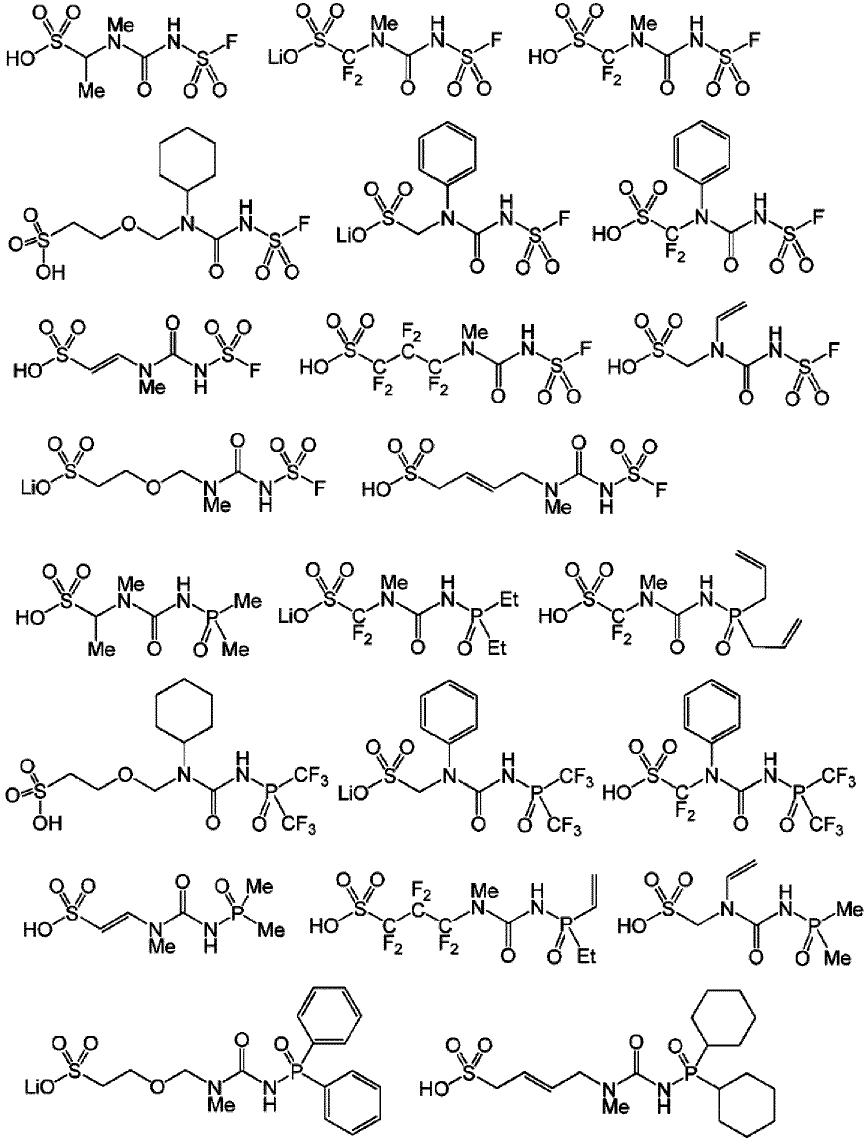

In the nonaqueous electrolyte solution according to the present disclosure, a lower limit of a content of the above Component (I) (hereafter, also described as "concentration of (I)") with respect to a total amount (100% by mass) of the nonaqueous electrolyte solution is preferably 0.01% by mass or more, more preferably 0.05% by mass or more, and further preferably 0.1% by mass or more. An upper limit of the concentration of (I) is preferably 10.0% by mass or less, more preferably 5.0% by mass or less, further preferably 4.0% by mass or less, and particularly preferably 2.5% by mass or less.

Setting the concentration of (I) to 0.01% by mass or more easily achieves an effect of improving cycle characteristics of a nonaqueous electrolyte solution battery using the nonaqueous electrolyte solution. On the other hand, setting the concentration of (I) to 10.0% by mass or less allows for suppressing an increase in viscosity of the nonaqueous electrolyte solution, and easily achieves the effect of preventing the increase in the resistance of the nonaqueous electrolyte solution battery using the nonaqueous electrolyte solution.

The content of the above Component (I) with respect to the total amount of the nonaqueous electrolyte solution is preferably 0.01% by mass to 5.0% by mass.

In the nonaqueous electrolyte solution according to the present disclosure, one type of compounds in Component (I) may be used alone, or two or more types of compounds may be mixed and used in any combination and any proportion according to an application.

The compound represented by Formula (1) can be produced by various methods. The method for producing the compound represented by Formula (1) is not limited.

For example, the compound represented by Formula (1) can be obtained by causing 2-hydroxyethanesulfonate or hydroxymethanesulfonate to react with fluorosulfonyl isocyanate, followed by reacting with lithium hydride.

The present disclosure also relates to the compound represented by the above Formula (1).

The above compound is suitably used as an additive in the nonaqueous electrolyte solution. That is, the present disclosure also relates to an additive for nonaqueous electrolyte solution represented by the above Formula (1).

### <(II) Solute>

The nonaqueous electrolyte solution according to the present disclosure contains a solute.

The solute is not limited, but is preferably an ionic salt, more preferably an ionic salt containing fluorine.

The solute is preferably, for example, an ionic salt containing a pair of at least one cation selected from the group consisting of alkali metal ions such as lithium ions and sodium ions, alkaline earth metal ions, and quaternary ammonium, and at least one anion selected from the group consisting of a hexafluorophosphate anion, a tetrafluoroborate anion, a perchlorate anion, a hexafluoroarsenate anion, a hexafluoroantimonate anion, a trifluoromethanesulfonate anion, a bis(trifluoromethanesulfonyl)imide anion, a bis(pentafluoroethanesulfonyl)imide anion, a (trifluoromethanesulfonyl)(pentafluoroethanesulfonyl)imide anion, a bis(fluorosulfonyl)imide anion, a (trifluoromethanesulfonyl)(fluorosulfonyl)imide anion, a (pentafluoroethanesulfonyl)(fluorosulfonyl)imide anion, and a tris(trifluoromethanesulfonyl)methide anion.

The solute is preferably at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlOz, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

One type of these solutes may be used alone, or two or more types thereof may be mixed and used in any combination and any proportion according to an application.

Among the above solutes, from the viewpoint of energy density, output characteristics, life, and the like of a nonaqueous electrolyte solution battery, the cation is preferably at least one selected from the group consisting of lithium, sodium, potassium, magnesium, and quaternary ammonium, and the anion is preferably at least one selected from the group consisting of a hexafluorophosphate anion, a tetrafluoroborate anion, a bis(trifluoromethanesulfonyl)imide anion, and a bis(fluorosulfonyl)imide anion.

A total amount of the solute (hereinafter, also described as "solute concentration") in the nonaqueous electrolyte solution according to the present disclosure is not limited, but a lower limit thereof is preferably 0.5 mol/L or more, more preferably 0.7 mol/L or more, and further preferably 0.9 mol/L or more. In addition, an upper limit of the solute concentration is preferably 5.0 mol/L or less, more preferably 4.0 mol/L or less, and further preferably 2.0 mol/L or less. Setting the solute concentration to 0.5 mol/L or more allows for reducing deterioration of the cycle characteristics and the output characteristics of the nonaqueous electrolyte solution battery due to deterioration in ionic conductivity, and setting the solute concentration to 5.0 mol/L or less allows for reducing the deterioration in ionic conductivity, and the deterioration in the cycle characteristics and the output characteristics of the nonaqueous electrolyte solution battery due to an increase in the viscosity of the nonaqueous electrolyte solution.

### <(III) Nonaqueous Organic Solvent>

A type of the nonaqueous organic solvent used in the nonaqueous electrolyte solution according to the present disclosure is not limited, and any nonaqueous organic solvent can be used.

The nonaqueous organic solvent is preferably at least one selected from the group consisting of a cyclic ester, a chain ester, a cyclic ether, a chain ether, a sulfone compound, a sulfoxide compound, and an ionic liquid.

Specifically, the nonaqueous organic solvent is preferably at least one selected from the group consisting of ethyl methyl carbonate (hereinafter, also described as "EMC"), dimethyl carbonate (hereinafter, also described as "DMC"), diethyl carbonate (hereinafter, also described as "DEC"), methyl propyl carbonate, ethyl propyl carbonate, methyl butyl carbonate, 2,2,2-trifluoroethyl methyl carbonate, 2,2,2-trifluoroethyl ethyl carbonate, 2,2,2-trifluoroethyl propyl carbonate, bis(2,2,2-trifluoroethyl) carbonate, 1,1,1,3,3,3-hexafluoro-1-propylmethyl carbonate, 1,1,1,3,3,3-hexafluoro-1-propylethyl carbonate, 1,1,1,3,3,3-hexafluoro-1-propylpropyl carbonate, bis(1,1,1,3,3,3-hexafluoro-1-propyl) carbonate, ethylene carbonate (hereinafter also described as "EC"), propylene carbonate (hereinafter, also described as "PC"), butylene carbonate, fluoroethylene carbonate (hereinafter, also described as "FEC"), difluoroethylene carbonate, methyl acetate, ethyl acetate, methyl propionate, ethyl propionate, methyl 2-fluoropropionate, ethyl 2-fluoropropionate, diethyl ether, dibutyl ether, diisopropyl ether, 1,2-dimethoxyethane, tetrahydrofuran, 2-methyltetrahydrofuran, furan, tetrahydropyran, 1,3-dioxane, 1,4-dioxane, N,N-dimethylformamide, acetonitrile, propionitrile, dimethyl sulfoxide, sulfolane, γ-butyrolactone, and γ-valerolactone.

In the present disclosure, an ionic liquid, which has a salt structure, may be used as the nonaqueous organic solvent.

The nonaqueous organic solvent is preferably at least one selected from the group consisting of a cyclic ester and a chain ester because the preferable solvent achieves excellent input and output characteristics at low temperatures.

The above nonaqueous organic solvent is preferably at least one selected from the group consisting of cyclic carbonates and chain carbonates because the preferable solvent achieves excellent cycle characteristics at a high temperature.

Preferably, the nonaqueous organic solvent contains a cyclic ester, and the cyclic ester is cyclic carbonate.

Specific examples of the above cyclic carbonate include EC, PC, butylene carbonate, and FEC, and at least one selected from the group consisting of EC, PC, and FEC is preferable.

Preferably, the nonaqueous organic solvent contains a chain ester, and the chain ester is chain carbonate.

Specific examples of the above chain carbonate include EMC, DMC, DEC, methyl propyl carbonate, ethyl propyl carbonate, 2,2,2-trifluoroethyl methyl carbonate, 2,2,2-trifluoroethyl ethyl carbonate, 1,1,1,3,3,3-hexafluoro-1-propylmethyl carbonate, and 1,1,1,3,3,3-hexafluoro-1-propylethyl carbonate, and at least one selected from the group consisting of EMC, DMC, DEC, and methyl propyl carbonate is preferable.

Specific examples of the above ester include methyl acetate, ethyl acetate, methyl propionate, ethyl propionate, methyl 2-fluoropropionate, and ethyl 2-fluoropropionate.

### <Another Additive>

An additive component to be commonly used in the nonaqueous electrolyte solution according to the present disclosure may be further added in any proportion as long as the gist of the present disclosure is not impaired.

Specific examples of another additive include compounds having an overcharge prevention effect, a negative electrode film-forming effect, and a positive electrode protective effect, such as cyclohexylbenzene, cyclohexylfluorobenzene, fluorobenzene, biphenyl, difluoroanisole, tert-butylbenzene, tert-amylbenzene, 2-fluorotoluene, 2-fluorobiphenyl, vinylene carbonate, dimethylvinylene carbonate, vinylethylene carbonate, fluoroethylene carbonate, trans-difluoroethylene carbonate, methyl propargyl carbonate, ethyl propargyl carbonate, dipropargyl carbonate, maleic anhydride, succinic anhydride, propanesultone, 1,3-propanesultone, 1,3-propenesultone, butanesultone, 1,3,2-dioxathiolane-2,2-dioxide, 4-propyl-1,3,2-dioxathiolane-2,2-dioxide, methylene methanedisulfonate, dimethylene methanedisulfonate, trimethylene methanedisulfonate, methyl methanesulfonate, 1,6-diisocyanatohexane, tris(trimethylsilyl)borate, succinonitrile, (ethoxy)pentafluorocyclotriphosphazene, lithium difluorobis(oxalato)phosphate, sodium difluorobis(oxalato)phosphate, potassium difluorobis(oxalato)phosphate, lithium difluorooxalatoborate, sodium difluorooxalatoborate, potassium difluorooxalatoborate, lithium bis(oxalato)borate, sodium bis(oxalato)borate, potassium bis(oxalato)borate, lithium tetrafluorooxalatophosphate, sodium tetrafluorooxalatophosphate, potassium tetrafluorooxalatophosphate, lithium tris(oxalato)phosphate, sodium tris(oxalato)phosphate, potassium tris(oxalato)phosphate, lithium difluorophosphate, sodium difluorophosphate, potassium difluorophosphate, lithium monofluorophosphate, sodium monofluorophosphate, potassium monofluorophosphate, lithium fluorosulfonate, sodium fluorosulfonate, potassium fluorosulfonate, lithium bis(difluorophosphoryl)imide, sodium bis(difluorophosphoryl)imide, potassium bis(difluorophosphoryl)imide, methanesulfonyl fluoride, ethenesulfonyl fluoride, and phenyl difluorophosphate.

The nonaqueous electrolyte solution according to the present disclosure may contain at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene. A content of the above additive in the nonaqueous electrolyte solution is preferably 0.01% by mass or more and 5.0% by mass or less with respect to the total amount of the nonaqueous electrolyte solution.

The nonaqueous electrolyte solution according to the present disclosure may contain a compound represented by the following Formula (3) as another additive.

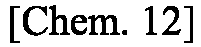

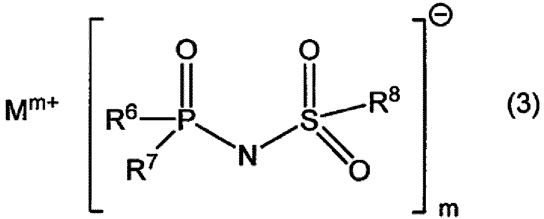

[In Formula (3), R⁶ to R⁸ are each independently a fluorine atom or an organic group selected from a linear alkyl group having 1 to 10 carbon atoms, a branched alkyl group having 3 to 10 carbon atoms, a linear alkoxy group having 1 to 10 carbon atoms, a branched alkoxy group having 3 to 10 carbon atoms, an alkenyl group having 2 to 10 carbon atoms, an alkenyloxy group having 2 to 10 carbon atoms, an alkynyl group having 2 to 10 carbon atoms, an alkynyloxy group having 2 to 10 carbon atoms, a cycloalkyl group having 3 to 10 carbon atoms, a cycloalkoxy group having 3 to 10 carbon atoms, a cycloalkenyl group having 3 to 10 carbon atoms, a cycloalkenyloxy group having 3 to 10 carbon atoms, an aryl group having 6 to 10 carbon atoms, and an aryloxy group having 6 to 10 carbon atoms, and the organic group may have a fluorine atom, an oxygen atom, or an unsaturated bond. Here, at least one of R⁶ to R⁸ is a fluorine atom.

M^{m+} represents an alkali metal cation, an alkaline earth metal cation, or an onium cation, and m represents the same integer as a valence of the corresponding cation.]

When the compound (salt having an imide anion) represented by Formula (3) has at least one P-F bond or S-F bond, the compound achieves excellent low-temperature characteristics. The larger number of P-F bonds and S-F bonds in the salt having the above imide anion equates to the more improvement in low-temperature characteristics, which is preferable, and in the salt having the imide anion represented by the above Formula (3), a compound in which all of R⁶ to R⁸ are fluorine atoms is more preferable.

In the salt having the imide anion represented by the above Formula (3), the preferable compound is a compound in which at least one of R⁶ to R⁸ is a fluorine atom, and at least one of R⁶ to R⁸ is selected from a hydrocarbon group having 6 or less carbon atoms and optionally having a fluorine atom.

In the salt having the imide anion represented by the above Formula (3), the preferable compound is a compound in which at least one of R⁶ to R⁸ is a fluorine atom, and at least one of R⁶ to R⁸ is selected from a methyl group, a methoxy group, an ethyl group, an ethoxy group, a propyl group, a propoxy group, a vinyl group, an allyl group, an allyloxy group, an ethynyl group, a 2-propynyl group, a 2-propynyloxy group, a phenyl group, a phenyloxy group, a 2,2-difluoroethyl group, a 2,2-difluoroethyloxy group, a 2,2,2-trifluoroethyl group, a 2,2,2-trifluoroethyloxy group, a 2,2,3,3-tetrafluoropropyl group, a 2,2,3,3-tetrafluoropropyloxy group, a 1,1,1,3,3,3-hexafluoroisopropyl group, and a 1,1,1,3,3,3-hexafluoroisopropyloxy group.

A counter cation M^{m+} of the salt having the imide anion represented by Formula (3) is preferably selected from the group consisting of lithium ion, sodium ion, potassium ion, and tetraalkylammonium ion.

In the above Formula (3), examples of the alkyl group and the alkoxy group represented by R⁶ to R⁸ include an alkyl group having 1 to 10 carbon atoms, a fluorine-containing alkyl group, and an alkoxy group derived from these groups, such as a methyl group, an ethyl group, a propyl group, an isopropyl group, a butyl group, a sec-butyl group, a tert-butyl group, a pentyl group, a 2,2-difluoroethyl group, 2,2,2-trifluoroethyl group, 2,2,3,3-tetrafluoropropyl group, and a 1,1,1,3,3,3-hexafluoroisopropyl group.

Examples of the alkenyl group and the alkenyloxy group include an alkenyl group having 2 to 10 carbon atoms such as a vinyl group, an allyl group, a 1-propenyl group, an isopropenyl group, a 2-butenyl group, and a 1,3-butadienyl group, fluorine-containing alkenyl groups, and alkenyloxy groups derived from these groups.

Examples of the alkynyl group and the alkynyloxy group include an alkynyl group having 2 to 10 carbon atoms such as an ethynyl group, a 2-propynyl group, and a 1,1-dimethyl-2-propynyl group, fluorine-containing alkynyl groups, and alkynyloxy groups derived from these groups.

Examples of the cycloalkyl group and the cycloalkoxy group include a cycloalkyl group having 3 to 10 carbon atoms such as a cyclopentyl group and a cyclohexyl group, fluorine-containing cycloalkyl groups, and cycloalkoxy groups derived from these groups.

Examples of the cycloalkenyl group and the cycloalkenyloxy group include a cycloalkenyl group having 3 to 10 carbon atoms such as a cyclopentenyl group and a cyclohexenyl group, fluorine-containing cycloalkenyl groups, and cycloalkenyloxy groups derived from these groups.

Examples of the aryl group and the aryloxy group include an aryl group having 6 to 10 carbon atoms such as a phenyl group, a tolyl group, and an xylyl group, fluorine-containing aryl groups, and aryloxy groups derived from these groups.

Specific examples and synthesis methods of the salt having the imide anion represented by Formula (3) include those described in WO2017/111143.

The content of another additive in the nonaqueous electrolyte solution is preferably 0.01% by mass or more and 8.0% by mass or less with respect to the total amount of the nonaqueous electrolyte solution.

When the content of the ionic salt exemplified as the solute is less than 0.5 mol/L, which is the lower limit of the suitable concentration of the solute, in the nonaqueous electrolyte solution, the ionic salt can exert a negative electrode film-forming effect and/or a positive electrode protective effect as "another additive". In this case, the content in the nonaqueous electrolyte solution is preferably 0.01% by mass to 5.0% by mass.

For example, when the nonaqueous electrolyte solution battery is a lithium ion battery, examples of the ionic salt include lithium hexafluorophosphate, lithium tetrafluoroborate, lithium trifluoromethanesulfonate, lithium bis(trifluoromethanesulfonyl)imide, lithium bis(fluorosulfonyl)imide, and lithium (trifluoromethanesulfonyl)(fluorosulfonyl)imide, and when the nonaqueous electrolyte solution battery is a sodium ion battery, examples of the ionic salt include sodium hexafluorophosphate, sodium tetrafluoroborate, sodium trifluoromethanesulfonate, sodium bis(trifluoromethanesulfonyl)imide, sodium bis(fluorosulfonyl)imide, and sodium (trifluoromethanesulfonyl)(fluorosulfonyl)imide.

An alkali metal salt other than the above solutes may be used as an additive.

Specific examples include carboxylates such as lithium acrylate, sodium acrylate, lithium methacrylate, and sodium methacrylate, and sulfuric acid ester salts such as lithium methyl sulfate, sodium methyl sulfate, lithium ethyl sulfate, and sodium ethyl sulfate.

From a viewpoint of improving durability (life) of a battery, when the nonaqueous electrolyte solution battery is a lithium ion battery, the nonaqueous electrolyte solution according to the present disclosure is preferable to contain, with respect to the total amount of the nonaqueous electrolyte solution, 0.01% by mass to 5.0% by mass of at least one selected from vinylene carbonate, fluoroethylene carbonate, lithium bis(oxalato)borate, lithium difluorooxalatoborate, lithium difluorobis(oxalato)phosphate, lithium tetrafluorooxalatophosphate, lithium bis(fluorosulfonyl)imide, lithium (difluorophosphoryl)(fluorosulfonyl)imide, lithium difluorophosphate, lithium fluorosulfonate, 1,3-propenesultone, 1,3-propanesultone, 1,3,2-dioxathiolane-2,2-dioxide, and 4-propyl-1,3,2-dioxathiolane-2,2-dioxide in the above another additive.

When the nonaqueous electrolyte solution battery is a sodium ion battery, the nonaqueous electrolyte solution according to the present disclosure is preferable to contain, with respect to the total amount of the nonaqueous electrolyte solution, 0.01% by mass to 5.0% by mass of at least one selected from vinylene carbonate, fluoroethylene carbonate, sodium bis(oxalato)borate, sodium difluorooxalatoborate, sodium difluorobis(oxalato)phosphate, sodium tetrafluorooxalatophosphate, sodium bis(fluorosulfonyl)imide, sodium (difluorophosphoryl)(fluorosulfonyl)imide, sodium difluorophosphate, sodium fluorosulfonate, 1,3-propenesultone, 1,3-propanesultone, 1,3,2-dioxathiolane-2,2-dioxide, and 4-propyl-1,3,2-dioxathiolane-2,2-dioxide.

In addition, the nonaqueous electrolyte solution according to the present disclosure can also contain a polymer, and as in the case of being used in a nonaqueous electrolyte solution battery referred to as a polymer battery, the nonaqueous electrolyte solution can be quasi-solidified with a gelling agent or a cross-linked polymer. A polymer solid electrolyte includes one containing the nonaqueous organic solvent as a plasticizer.

The above polymer is not limited as long as the polymer is an aprotic polymer capable of dissolving the compound represented by the above Formula (1), the above solute, and the above another additive. Examples of the above polymer include polymers having polyethylene oxide as a main chain or a side chain, homopolymers or copolymers of polyvinylidene fluoride, methacrylic acid ester polymers, and polyacrylonitrile. When a plasticizer is added to these polymers, an aprotic nonaqueous organic solvent is preferable among the above nonaqueous organic solvents.

### [2. Nonaqueous Electrolyte Solution Battery]

The nonaqueous electrolyte solution battery according to the present disclosure includes, at least, the nonaqueous electrolyte solution according to the present disclosure described above, a negative electrode, and a positive electrode. Furthermore, a separator, an exterior body, and the like are preferably included.

The negative electrode is not limited, but preferably uses a material capable of reversibly intercalating and deintercalating alkali metal ions such as lithium ions and sodium ions, or alkaline earth metal ions.

For example, in the case of the lithium ion secondary battery in which cations are mainly lithium, a negative electrode active material constituting the negative electrode is capable of doping and dedoping lithium ions. Examples of the negative electrode active material includes a material containing at least one selected from, for example, a carbon material in which a d value of a lattice plane (002 plane) in X-ray diffraction is 0.340 nm or less, a carbon material in which the d value of the lattice plane (002 plane) in X-ray diffraction exceeds 0.340 nm, oxides of one or more metal selected from Si, Sn, and Al, one or more metals selected from Si, Sn, and Al, alloys containing these metals, alloys of these metals and lithium, or alloys of the above alloys and lithium, and lithium titanium oxide. One of these negative electrode active materials can be used alone, or two or more thereof can be used in combination. Lithium metal, metal nitrides, tin compounds, conductive polymers, and the like may also be used.

For example, in a case of a sodium ion secondary battery in which cations are mainly sodium, as the negative electrode active material constituting the negative electrode, sodium metal, an alloy of sodium metal and other metals such as tin, an intermetallic compound, various carbon materials such as hard carbon, a metal oxide such as titanium oxide, a metal nitride, (elemental) tin, a tin compound, activated carbon, a conductive polymer, and the like are used. In addition to these, (elemental) phosphorus such as red phosphorus and black phosphorus, phosphorus compounds such as Co-P, Cu-P, Sn-P, Ge-P, and Mo-P, (elemental) antimony, antimony compounds such as Sb/C and Bi-Sb, and the like are used. One type of these negative electrode active materials may be used alone, or two or more types thereof may be used in combination.

The positive electrode is not limited, but preferably uses a material capable of reversibly intercalating and deintercalating alkali metal ions such as lithium ions and sodium ions, or alkaline earth metal ions.

For example, when the cation is lithium, lithium-containing transition metal composite oxides such as LiCoO₂, LiNiOz, LiMnO₂, and LiMn₂O₄, these lithium-containing transition metal composite oxides including a mixture of a plurality of transition metals such as Co, Mn, and Ni, these lithium-containing transition metal composite oxides in which a part of the transition metals is substituted by a metal other than the transition metals, phosphate compounds of transition metals such as LiFePO₄, LiCoPO₄, and LiMnPO₄ referred to as olivine, oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like can be used as a positive electrode material.

Specific examples include Li[Ni_{1/3}Mn_{1/3}Co_{1/3}]O₂, Li[Ni_{0.45}Mn_{0.35}Co_{0.2}]O₂, Li[Ni_{0.5}Mn_{0.3}Co_{0.2}]O₂, Li[Ni_{0.6}Mn_{0.2}Co_{0.2}]O₂, Li[Ni_{0.8}Mn_{0.1}Co_{0.1}]O₂, Li[Ni_{0.49}Mn_{0.3}Co_{0.2}Zr_{0.01}]O₂, Li[Ni_{0.49}Mn_{0.3}Co_{0.2}Mg_{0.01}]O₂, LiNi_{0.8}Co_{0.2}O₂, LiNi_{0.85}Co_{0.10}Al_{0.05}O₂, LiNi_{0.87}Co_{0.10}Al_{0.03}O₂, LiNi_{0.90}Co_{0.07}Al_{0.03}O₂, LiNi_{0.6}Co_{0.3}Al_{0.1}O₂, and LiMn_{1.5}Ni_{0.5}O₄.

For example, when the cation is sodium, sodium-containing transition metal composite oxides such as NaCrO₂, NaFe_{0.5}Co_{0.5}O₂, NaFe_{0.4}Mn_{0.3}Ni_{0.3}O₂, NaNi_{0.5}Ti_{0.3}Mn_{0.2}O₂, NaNi_{1/3}Ti_{1/3}Mn_{1/3}O₂, NaNi_{0.33}Ti_{0.33}Mn_{0.16}Mg_{0.17}O₂, Na_{2/3}Ni_{1/3}Ti_{1/6}Mn_{1/2}O₂, and Na_{2/3}Ni_{1/3}Mn_{2/3}O₂, these sodium-containing transition metal composite oxides including a mixture of a plurality of transition metals such as Co, Mn, and Ni, these sodium-containing transition metal composite oxides in which a part of the transition metals is substituted with a metal other than the transition metals, polyanion type compounds such as NaFePO₄, NaVPO₄F, Na₃V₂(PO₄)₃, and Na₂Fe₂(SO₄)₃, sodium salts of a Prussian Blue analogues represented by a composition formula NaₐM_{b}[Fe(CN)₆]_{c} (M = Cr, Mn, Fe, Co, Ni, Cu, or Zn, 0 ≤ a ≤ 2, 0.5 ≤ b ≤ 1.5, 0.5 ≤ c ≤ 1.5), oxides such as TiO₂, V₂O₅, and MoO₃, sulfides such as TiS₂ and FeS, conductive polymers such as polyacetylene, polyparaphenylene, polyaniline, and polypyrrole, activated carbon, radical-generating polymers, carbon materials, and the like can be used as a positive electrode material (positive electrode active material).

As the positive electrode and negative electrode materials, an electrode sheet obtained by adding acetylene black, ketjen black, carbon fiber, or graphite as a conductive material, adding polytetrafluoroethylene, polyvinylidene fluoride, SBR resin, or the like as a binder to the materials and molding the mixture into a sheet shape, can be used.

As a separator for preventing contact between the positive electrode and the negative electrode, a nonwoven fabric or porous sheet made of polyolefin, polyethylene, paper, glass fiber, or the like is used.

An electrochemical device having a shape such as a coin shape, a cylindrical shape, a square shape, or an aluminum laminate sheet shape is assembled based on the above elements.

### EXAMPLES

The present disclosure will be described in more detail below with reference to Examples, but the present disclosure is not limited by these descriptions.

### <Synthesis Example-1>

### Synthesis of Compound (a-1)

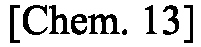

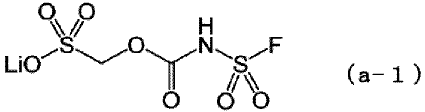

After 100 g of acetonitrile (MeCN) and 1.2 g of Li hydroxymethanesulfonic acid were put into a 50 ml eggplant flask, 1.7 g of chlorosulfonyl isocyanate was slowly added. After an internal temperature of the flask decreased to a room temperature, 0.5 g of sodium fluoride was added. The reaction solution was filtered, and concentration was carried out to obtain 2.0 g of Compound (a-1) ((yield: 76%).
¹H NMR(CD₃CN) σ_{H} 11.49 ppm, 4.88 ppm,
¹⁹F NMR(CD₃CN) σ_{F} 51.0 ppm.

### <Synthesis Example-2>

### Synthesis of Compound (a-2)

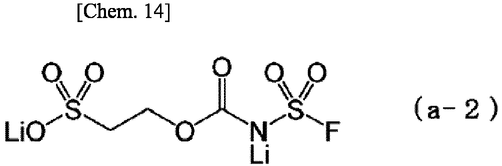

After 100 g of MeCN and 1.3 g of Li 2-hydroxyethanesulfonic acid were put in the 50 ml eggplant flask, 1.5 g of fluorosulfonyl isocyanate was slowly added. After an internal temperature of the flask decreased to a room temperature, 0.08 g of lithium hydride was added. After foaming ceased, concentration was carried out to obtain 2.5 g of Compound (a-2) (yield: 95%).
¹H NMR(CD₃CN) σ_{H} 4.38 ppm, 3.26 ppm,
¹⁹F NMR(CD₃CN) σ_{F} 49.1 ppm.

### <Synthesis Example-3>

### Synthesis of Compound (a-3)

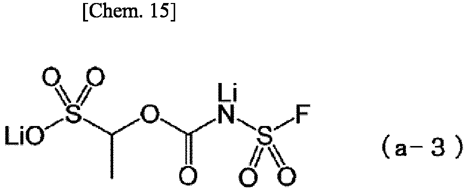

After 100 g of MeCN and 1.3 g of Li 1-hydroxyethanesulfonic acid were put in the 50 ml eggplant flask, 1.5 g of fluorosulfonyl isocyanate was slowly added. After an internal temperature of the flask decreased to a room temperature, 0.08 g of lithium hydride was added. After foaming ceased, concentration was carried out to obtain 2.4 g of Compound (a-3) (yield: 92%).
¹H NMR(CD₃CN) σ_{H} 5.43 ppm, 1.48 ppm,
¹⁹F NMR(CD₃CN) σ_{F} 49.0 ppm.

### <Synthesis Example-4>

### Synthesis of Compound (a-4)

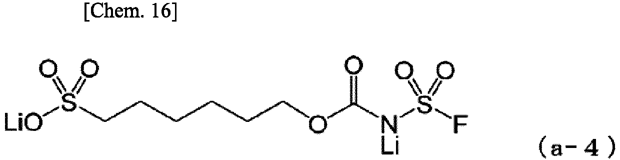

After 100 g of MeCN and 1.9 g of Li 6-hydroxyhexane sulfonate were put in the 50 ml eggplant flask, 1.5 g of fluorosulfonyl isocyanate was slowly added. After an internal temperature of the flask decreased to a room temperature, 0.08 g of lithium hydride was added. After foaming ceased, concentration was carried out to obtain 2.9 g of Compound (a-4) (yield: 90%).
¹H NMR(CD₃CN) σ_{H} 4.45 ppm, 3.31 ppm, 1.57 ppm to 0.94 ppm,
¹⁹F NMR(CD₃CN) σ_{F} 49.3 ppm.

### <Synthesis Example-5>

### Synthesis of Compound (a-5)

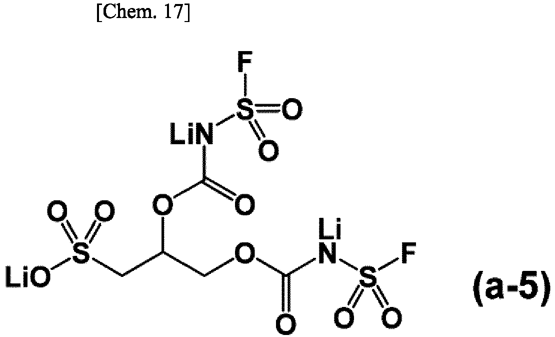

After 100 g of MeCN and 1.6 g of Li 2,3-dihydroxypropanesulfonic acid were put in the 50 ml eggplant flask, 1.5 g of fluorosulfonyl isocyanate was slowly added. After an internal temperature of the flask decreased to a room temperature, 0.08 g of lithium hydride was added. After foaming ceased, concentration was carried out to obtain 3.4 g of Compound (a-5) (yield: 80%).
¹H NMR(CD₃CN) σ_{H} 5.38 ppm, 4.55 ppm, 4.43 ppm, 4.10 ppm, 3.40 ppm, 3.21 ppm,
¹⁹F NMR(CD₃CN) σ_{F} 51.6 ppm, 51.1 ppm.

The following Compounds a-1 (Na) to a-5 (Na) were obtained by subjecting the above Compounds (a-1) to (a-5) to a cation exchange reaction, respectively. Compounds a-1 (Na) to a-5 (Na) can also be obtained by using sodium hydroxyalkanesulfonate corresponding to each raw material, allowing it to react with fluorosulfonyl isocyanate, and neutralizing the resultant product with sodium hydride.

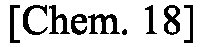

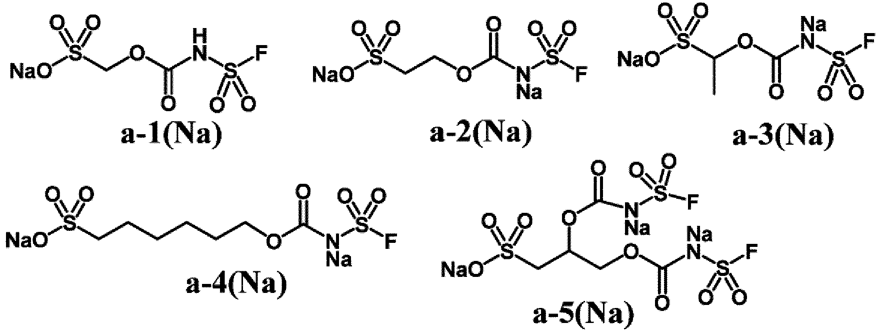

### [Preparation of Nonaqueous Electrolyte Solutions according to Examples and Comparative Examples]

### <Comparative Example 1-1>

### (Preparation of LiPF₆ Solution)

EC, FEC, EMC, and DMC were mixed in a volume ratio of EC:FEC:EMC:DMC = 25:5:45:25 in a glove box with a dew point of -60°C or lower (Component (III)). After that, LiPF₆ (Component (II)) was added such that LiPF₆ had a concentration of 1.0 mol/L, with an internal temperature at 40°C or lower kept. Then, the mixture was stirred, and completely dissolved to obtain a LiPF₆ solution. This LiPF₆ solution was defined as Comparative Nonaqueous Electrolyte Solution 1-1.

### <Example 1-1>

### (Preparation of Nonaqueous Electrolyte Solution 1-1)

EC, FEC, EMC, and DMC were mixed in a volume ratio of EC:FEC:EMC:DMC = 25:5:45:25 in a glove box with a dew point of -60°C or lower (Component (III)). After that, LiPF₆ (Component (II)) was added such that LiPF₆ had a concentration of 1.0 mol/L, with an internal temperature at 40°C or lower kept. Compound (a-1) (Component (I)) corresponding to the compound represented by Formula (1) was added such that Compound (a-1) had a concentration of 0.05% by mass with respect to the total amount of the nonaqueous electrolyte solution. Then, the mixture was stirred and dissolved for 1 hour to obtain Nonaqueous Electrolyte Solution 1-1 according to Example 1-1.

### <Examples 1-2 and 1-15, Comparative Examples 1-2 and 1-3>

### (Preparation of Nonaqueous Electrolyte Solutions 1-2 and 1-15 and Comparative Nonaqueous Electrolyte Solutions 1-2 and 1-3)

Except that the type and content of Component (I) (or comparative compound) were changed as described in Table 1, Nonaqueous Electrolyte Solutions 1-2 to 1-15 and Comparative Nonaqueous Electrolyte Solutions 1-2 and 1-3 were obtained in the same way as in the preparation of Nonaqueous Electrolyte Solution 1-1. Compound (X) was purchased from Tokyo Chemical Industry Co., Ltd. (product code H0597). Compound (Y) was synthesized by the same method as that disclosed in WO2014/073378.

Structures of Compounds (X) and (Y) used in Comparative Examples are shown below.

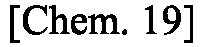

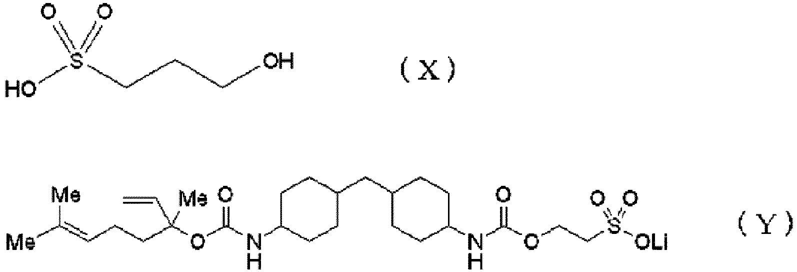

### <Examples 2-1 to 2-3, Comparative Examples 2-1 to 2-3>

### (Preparation of Nonaqueous Electrolyte Solutions 2-1 to 2-3 and Comparative Nonaqueous Electrolyte Solutions 2-1 to 2-3)

Furthermore, except that vinylene carbonate (VC) as another additive was added and dissolved such that VC had a concentration described in Table 2, Nonaqueous Electrolyte Solutions 2-1 to 2-3 and Comparative Nonaqueous Electrolyte Solutions 2-1 to 2-3 were respectively obtained in the same way as in the preparation of Nonaqueous Electrolyte Solutions 1-4, 1-9, and 1-11 and Comparative Nonaqueous Electrolyte Solutions 1-1 to 1-3.

### <Examples 3-1 to 3-3, Comparative Examples 3-1 to 3-3>

### (Preparation of Nonaqueous Electrolyte Solutions 3-1 to 3-3 and Comparative Nonaqueous Electrolyte Solutions 3-1 to 3-3)

Except that VC was changed to lithium bis(oxalato)borate (BOB), Nonaqueous Electrolyte Solutions 3-1 to 3-3 and Comparative Nonaqueous Electrolyte Solutions 3-1 to 3-3 were respectively obtained in the same way as in the preparation of Nonaqueous Electrolyte Solutions 2-1 to 2-3 and Comparative Nonaqueous Electrolyte Solutions 2-1 to 2-3.

### <Examples 4-1 to 4-3, Comparative Examples 4-1 to 4-3>

### (Preparation of Nonaqueous Electrolyte Solutions 4-1 to 4-3 and Comparative Nonaqueous Electrolyte Solutions 4-1 to 4-3)

Except that VC was changed to lithium difluorobis(oxalato)phosphate (DFBOP), Nonaqueous Electrolyte Solutions 4-1 to 4-3 and Comparative Nonaqueous Electrolyte Solutions 4-1 to 4-3 were respectively obtained in the same way as the preparation of Nonaqueous Electrolyte Solutions 2-1 to 2-3 and Comparative Nonaqueous Electrolyte Solutions 2-1 to 2-3.

### <Examples 5-1 to 5-3, Comparative Examples 5-1 to 5-3>

### (Preparation of Nonaqueous Electrolyte Solutions 5-1 to 5-3 and Comparative Nonaqueous Electrolyte Solutions 5-1 to 5-3)

Except that VC was changed to lithium tetrafluorooxalatophosphate (TFOP), Nonaqueous Electrolyte Solutions 5-1 to 5-3 and Comparative Nonaqueous Electrolyte Solutions 5-1 to 5-3 were respectively obtained in the same way as the preparation of Nonaqueous Electrolyte Solutions 2-1 to 2-3 and Comparative Nonaqueous Electrolyte Solutions 2-1 to 2-3.

### <Examples 6-1 to 6-3, Comparative Examples 6-1 to 6-3>

### (Preparation of Nonaqueous Electrolyte Solutions 6-1 to 6-3 and Comparative Nonaqueous Electrolyte Solutions 6-1 to 6-3)

Except that VC was changed to lithium bis(fluorosulfonyl)imide (FSI), Nonaqueous Electrolyte Solutions 6-1 to 6-3 and Comparative Nonaqueous Electrolyte Solutions 6-1 to 6-3 were respectively obtained in the same way as the preparation of Nonaqueous Electrolyte Solutions 2-1 to 2-3 and Comparative Nonaqueous Electrolyte Solutions 2-1 to 2-3.

### <Examples 7-1 to 7-3, Comparative Examples 7-1 to 7-3>

### (Preparation of Nonaqueous Electrolyte Solutions 7-1 to 7-3 and Comparative Nonaqueous Electrolyte Solutions 7-1 to 7-3)

Except that VC was changed to lithium difluorophosphate (DFP), Nonaqueous Electrolyte Solutions 7-1 to 7-3 and Comparative Nonaqueous Electrolyte Solutions 7-1 to 7-3 were respectively obtained in the same way as the preparation of Nonaqueous Electrolyte Solutions 2-1 to 2-3 and Comparative Nonaqueous Electrolyte Solutions 2-1 to 2-3.

### <Examples 8-1 to 8-3, Comparative Examples 8-1 to 8-3>

### (Preparation of Nonaqueous Electrolyte Solutions 8-1 to 8-3 and Comparative Nonaqueous Electrolyte Solutions 8-1 to 8-3)

Except that VC was changed to lithium fluorosulfonate (FS), Nonaqueous Electrolyte Solutions 8-1 to 8-3 and Comparative Nonaqueous Electrolyte Solutions 8-1 to 8-3 were respectively obtained in the same way as the preparation of Nonaqueous Electrolyte Solutions 2-1 to 2-3 and Comparative Nonaqueous Electrolyte Solutions 2-1 to 2-3.

### <Example 17-1>

### (Preparation of Nonaqueous Electrolyte Solution 9-1)

PC, EC, FEC, and EMC were mixed in a volume ratio of PC:EC:FEC:EMC = 20:10:2:68 in a glove box with a dew point of -60°C or lower (Component (III)). After that, NaPF₆ (Component (II)) was added such that NaPF₆ had a concentration of the 1.0 mol/L, with the internal temperature at 40°C or lower kept. Compound (a-1 (Na)) (Component (I)) corresponding to the compound represented by Formula (1) was added such that Compound (a-1 (Na)) had a concentration of 0.5% by mass with respect to the total amount of the nonaqueous electrolyte solution. The mixture was stirred and dissolved for 1 hour to obtain Nonaqueous Electrolyte Solution 9-1 according to Example 17-1.

### <Examples 17-2 to 17-10, Comparative Examples 17-1 and 17-2>

### (Preparation of Nonaqueous Electrolyte Solutions 9-2 to 9-10 and Comparative Nonaqueous Electrolyte Solutions 9-1 and 9-2)

Except that the type and content of Component (I) (or comparative compound) were changed as described in Table 17, Nonaqueous Electrolyte Solutions 9-2 to 9-15 and Comparative Nonaqueous Electrolyte Solutions 9-1 and 9-2 were obtained in the same way as in the preparation of Nonaqueous Electrolyte Solution 9-1.

### <Examples 18-1 to 18-3, Comparative Examples 18-1 and 18-2>

### (Preparation of Nonaqueous Electrolyte Solutions 10-1 to 10-3 and Comparative Nonaqueous Electrolyte Solutions 10-1 and 10-2)

Furthermore, except that VC as another additive was added to a concentration described in Table 18 and dissolved, Nonaqueous Electrolyte Solutions 10-1 to 10-3 and Comparative Nonaqueous Electrolyte Solutions 10-1 and 10-2 were respectively obtained in the same way as in the preparation of Nonaqueous Electrolyte Solutions 9-1, 9-3, and 9-5 and Comparative Nonaqueous Electrolyte Solutions 9-1 and 9-2.

### <Examples 19-1 to 19-3, Comparative Examples 19-1 and 19-2>

### (Preparation of Nonaqueous Electrolyte Solutions 11-1 to 11-3 and Comparative Nonaqueous Electrolyte Solutions 11-1 and 11-2)

Except that VC was changed to sodium fluorosulfate (NaSO₃F) and sodium fluorosulfate was added and dissolved such that it had a concentration described in Table 19, Nonaqueous Electrolyte Solutions 11-1 to 11-3 and Comparative Nonaqueous Electrolyte Solutions 11-1and 11-2 were respectively obtained in the same way as the preparation of Nonaqueous Electrolyte Solutions 10-1 to 10-3 and Comparative Nonaqueous Electrolyte Solutions 10-1 and 10-2.

### <Examples 20-1 to 20-3, Comparative Examples 20-1 and 20-2>

### (Preparation of Nonaqueous Electrolyte Solutions 12-1 to 12-3 and Comparative Nonaqueous Electrolyte Solutions 12-1 and 12-2)

Except that VC was changed to sodium tetrafluorooxalatophosphate (TFOP-Na) and sodium tetrafluorooxalatophosphate was added and dissolved such that it had a concentration described in Table 20, Nonaqueous Electrolyte Solutions 12-1 to 12-3 and Comparative Nonaqueous Electrolyte Solutions 12-1 and 12-2 were respectively obtained in the same way as the preparation of Nonaqueous Electrolyte Solutions 10-1 to 10-3 and Comparative Nonaqueous Electrolyte Solutions 10-1 and 10-2.

### <Examples 21-1 to 21-3, Comparative Examples 21-1 and 21-2>

### (Preparation of Nonaqueous Electrolyte Solutions 13-1 to 13-3 and Comparative Nonaqueous Electrolyte Solutions 13-1 and 13-2)

Except that VC was changed to sodium difluorophosphate (DFP-Na) and sodium difluorophosphate was added and dissolved such that it had a concentration described in Table 21, Nonaqueous Electrolyte Solutions 13-1 to 13-3 and Comparative Nonaqueous Electrolyte Solutions 13-1 and 13-2 were respectively obtained in the same way as the preparation of Nonaqueous Electrolyte Solutions 10-1 to 10-3 and Comparative Nonaqueous Electrolyte Solutions 10-1 and 10-2.

In the following Tables 1 to 21, the content of Component (I) (or comparative compound) represents the concentration with respect to the total amount of nonaqueous electrolyte solution. The content of another additive represents the concentration with respect to the total amount of the nonaqueous electrolyte solution.

### [Production of Nonaqueous Electrolyte Solution Battery]

### (Production of Lithium Ion Battery Positive Electrode: NCM622 Positive Electrode)

LiNi_{0.6}Co_{0.2}Mn_{0.2}O₂ (90% by mass) as a positive electrode active material, acetylene black (5% by mass) as a conductive agent, and polyvinylidene fluoride (5% by mass) (hereinafter, also referred to as PVDF) as a binder were mixed, N-methyl-2-pyrrolidone as a solvent was further added such that N-methyl-2-pyrrolidone is in a content of 55% by mass with respect to a total mass of the positive electrode active material, the conductive agent, and the binder, and a slurry solution was prepared. The slurry solution was applied onto an aluminum foil serving as a positive electrode current collector and dried at 150°C for 12 hours to obtain a test NCM622 positive electrode in which a positive electrode active material layer was formed on the current collector.

### (Production of Lithium Ion Battery Positive Electrode: NCM811 Positive Electrode)

LiNi_{0.8}Co_{0.1}Mn_{0.1}O₂ (92% by mass) as a positive electrode active material, acetylene black (4.5% by mass) as a conductive agent, and PVDF (3.5% by mass) as a binder were mixed, N-methyl-2-pyrrolidone as a solvent was further added such that N-methyl-2-pyrrolidone is in a content of 55% by mass with respect to a total mass of the positive electrode active material, the conductive agent, and the binder, and a slurry solution was prepared. The slurry solution was applied onto an aluminum foil serving as a positive electrode current collector and dried at 150°C for 12 hours to obtain a test NCM811 positive electrode in which a positive electrode active material layer was formed on the current collector.

### (Production of Sodium Ion Battery Positive Electrode: NaNi_{0.5}Ti_{0.3}Mn_{0.2}O₂ Positive Electrode)

NaNi_{0.5}Ti_{0.3}Mn_{0.2}O₂ (90% by mass) as a positive electrode active material, acetylene black (5% by mass) as a conductive agent, and PVDF (5% by mass) as a binder were mixed, N-methyl-2-pyrrolidone as a solvent was further added such that N-methyl-2-pyrrolidone is in a content of 50% by mass with respect to a total mass of the positive electrode active material, the conductive agent, and the binder, and a slurry solution was prepared. The slurry solution was applied onto an aluminum foil serving as a positive electrode current collector and dried at 150°C for 12 hours to obtain a test NaNi_{0.5}Ti_{0.3}Mn_{0.2}O₂ positive electrode in which a positive electrode active material layer was formed on the current collector.

### (Production of Natural Graphite Negative Electrode)

Natural graphite powder (92% by mass), conductive material (3% by mass) (HS-100 manufactured by Denka), carbon nanofiber (2% by mass) (VGCF manufactured by Showa Denko), styrene-butadiene rubber (2% by mass), sodium carboxymethylcellulose (1% by mass), and water were mixed, and a slurry solution was prepared. The slurry solution was applied onto a copper foil serving as a negative electrode current collector and dried at 100°C for 12 hours to obtain a test natural graphite negative electrode in which a negative electrode active material layer was formed on the current collector.

### (Production of Silicon-Containing Graphite Negative Electrode)

To 85% by mass of artificial graphite powder, 7% by mass of nanosilicon, 3% by mass of conductive material (HS-100), 2% by mass of carbon nanofiber (VGCF), 2% by mass of styrene-butadiene rubber, 1% by mass of sodium carboxymethylcellulose, and water were mixed, and a slurry solution was prepared. The slurry solution was applied onto a copper foil serving as a negative electrode current collector and dried at 100°C for 12 hours to obtain a test silicon-containing graphite negative electrode in which a negative electrode active material layer was formed on the current collector.

### (Production of Hard Carbon Negative Electrode)

Hard carbon powder (90% by mass) (manufactured by Kureha, Carbotron P) as a negative electrode active material and PVDF (10% by mass) as a binder were mixed, N-methyl pyrrolidone as a solvent was further added such that N-methyl pyrrolidone is in a content of 50% by mass with respect to a total mass of the negative electrode active material and the binder, and a slurry solution was prepared. The slurry solution was applied onto an aluminum foil serving as a negative electrode current collector and dried at 150°C for 12 hours to obtain a test hard carbon negative electrode in which a negative electrode active material layer was formed on the current collector.

### (Production of Nonaqueous Electrolyte Solution Battery)

In an argon atmosphere with a dew point of -50°C or lower, the test NCM622 positive electrode and the test natural graphite negative electrode were placed through a polyethylene separator impregnated with a test electrolyte solution, and a nonaqueous electrolyte solution battery (lithium ion battery) with an aluminum laminate exterior was assembled. The nonaqueous electrolyte solution used those described in Tables 1 to 8.

In Examples 9-1 to 9-15, Comparative Examples 9-1 to 9-3, Examples 10-1 to 10-3, Comparative Examples 10-1 to 10-3, Examples 11-1 to 11-3, Comparative Examples 11-1 to 11-3, Examples 12-1 to 12-3, Comparative Examples 12-1 to 12-3, Examples 13-1 to 13-3, Comparative Examples 13-1 to 13-3, Examples 14-1 to 14-3, Comparative Examples 14-1 to 14-3, Examples 15-1 to 15-3, Comparative Examples 15-1 to 15-3, Examples 16-1 to 16-3, and Comparative Examples 16-1 to 16-3, NCM811 as the positive electrode and silicon-containing graphite as the negative electrode were used to produce a nonaqueous electrolyte solution battery (lithium ion battery) in the same manner. The nonaqueous electrolyte solution used those described in Tables 9 to 16.

In Examples 17-1 to 17-10, Comparative Examples 17-1 and 17-2, Examples 18-1 to 18-3, Comparative Examples 18-1 and 18-2, Examples 19-1 to 19-3, Comparative Examples 19-1 and 19-2, Examples 20-1 to 20-3, Comparative Examples 20-1 and 20-2, Examples 21-1 to 21-3, and Comparative Examples 21-1 and 21-2, the NaNi_{0.5}Ti_{0.5}Mn_{0.2}O₂ positive electrode as the positive electrode and the hard carbon negative electrode as the negative electrode were used to produce a nonaqueous electrolyte solution battery (sodium ion battery) in the same manner. The nonaqueous electrolyte solution used those described in Tables 17 to 21.

### [Evaluation] -Lithium Ion Battery: Initial Charge and Discharge-

The produced nonaqueous electrolyte solution battery was put in a 25°C constant temperature bath and, in this state, connected to a charge and discharge device. Charge was performed at 3 mA until 4.3 V. After 4.3 V was maintained for 1 hour, discharge was performed at 6 mA to 2.5 V. This was defined as one charge and discharge cycle, and three cycles in total of charge and discharge were performed to stabilize the battery.

### -Sodium Ion Battery: Initial Charge and Discharge-

The produced nonaqueous electrolyte solution battery was put in a 25°C constant temperature bath and, in this state, connected to a charge and discharge device. Charge was performed at 3 mA until 4.1 V. After 4.1 V was maintained for 1 hour, discharge was performed at 6 mA to 1.5 V. This was defined as one charge and discharge cycle, and three cycles in total of charge and discharge were performed to stabilize the battery.

### [Lithium Ion Battery: High-Temperature Cycle Characteristics Evaluation]

The nonaqueous electrolyte solution battery stabilized by performing the initial charge and discharge described above was subjected to a charge and discharge test at an environmental temperature of 60°C, and high-temperature cycle characteristics were evaluated. The charge and discharge cycle was repeated at a current value of 30 mA by using a constant current constant voltage method with an upper limit charge voltage of 4.3 V and a lower limit discharge voltage of 2.5 V. A degree of deterioration of the nonaqueous electrolyte solution battery was evaluated based on a discharge capacity retention rate at a 700th cycle in the charge and discharge test at the environmental temperature of 60°C. The "discharge capacity retention rate after high-temperature cycle" expressed as the discharge capacity retention rate at the 700th cycle was determined by the following formula. The discharge capacity of the first cycle in the charge and discharge test at the environmental temperature of 60°C was defined as an initial discharge capacity. The discharge capacity retention rate after high-temperature cycle (%) = discharge capacity at 700th cycle/initial discharge capacity) x 100

### [Sodium Ion Battery: High-Temperature Cycle Characteristics Evaluation]

Except that the upper limit charge voltage was changed to 4.1 V and the lower limit discharge voltage was changed to 1.5 V, an evaluation was performed in the same manner as in the lithium ion battery.

### [Lithium Ion Battery: Resistance Measurement After High-Temperature Cycle Test]

The nonaqueous electrolyte solution battery after the high-temperature cycle test described above was charged to 4.3 V at 25°C and 6 mA, and then a resistance value was measured by impedance measurement in a -20°C environment.

### [Sodium Ion Battery: Resistance Measurement After High-Temperature Cycle Test]

Except that the charge voltage was changed to 4.1 V, an evaluation was performed in the same manner as the lithium ion battery.

In each of Tables 1 to 21, a value of the resistance after high-temperature cycle test and the discharge capacity retention rate after high-temperature cycle of Comparative Example using the comparative nonaqueous electrolyte solution to which neither Component (I) nor a comparative compound was added (Comparative Example 1-1 in Table 1, Comparative Example 2-1 in Table 2, Comparative Example 3-1 in Table 3, Comparative Example 4-1 in Table 4, Comparative Example 5-1 in Table 5, Comparative Example 6-1 in Table 6, Comparative Example 7-1 in Table 7, Comparative Example 8-1 in Table 8, Comparative Example 9-1 in Table 9, Comparative Example 10-1 in Table 10, Comparative Example 11-1 in Table 11, Comparative Example 12-1 in Table 12, Comparative Example 13-1 in Table 13, Comparative Example 14-1 in Table 14, Comparative Example 15-1 in Table 15, Comparative Example 16-1 in Table 16, Comparative Example 17-1 in Table 17, Comparative Example 18-1 in Table 18, Comparative Example 19-1 in Table 19, Comparative Example 20-1 in Table 20, and Comparative Example 21-1 in Table 21) were respectively shown as 100, and an evaluation result in each of Examples and Comparative Examples was shown as a relative value.

**[Table 1]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 1-1 | Nonaqueous Electrolyte Solution 1-1 | (a-1) | 0.05 | - | - | 99 | 102 |
| Example 1-2 | Nonaqueous Electrolyte Solution 1-2 | (a-1) | 0.1 | - | - | 98 | 105 |
| Example 1-3 | Nonaqueous Electrolyte Solution 1-3 | (a-1) | 0.5 | - | - | 95 | 107 |
| Example 1-4 | Nonaqueous Electrolyte Solution 1-4 | (a-1) | 1.0 | - | - | 84 | 117 |
| Example 1-5 | Nonaqueous Electrolyte Solution 1-5 | (a-1) | 2.0 | - | - | 73 | 120 |
| Example 1-6 | Nonaqueous Electrolyte Solution 1-6 | (a-1) | 3.0 | - | - | 80 | 116 |
| Example 1-7 | Nonaqueous Electrolyte Solution 1-7 | (a-1) | 5.0 | - | - | 93 | 103 |
| Example 1-8 | Nonaqueous Electrolyte Solution 1-8 | (a-2) | 0.5 | - | - | 90 | 109 |
| Example 1-9 | Nonaqueous Electrolyte Solution 1-9 | (a-2) | 1.0 | - | - | 77 | 121 |
| Example 1-10 | Nonaqueous Electrolyte Solution 1-10 | (a-3) | 0.5 | - | - | 96 | 104 |
| Example 1-11 | Nonaqueous Electrolyte Solution 1-11 | (a-3) | 1.0 | - | - | 83 | 113 |
| Example 1-12 | Nonaqueous Electrolyte Solution 1-12 | (a-4) | 0.5 | - | - | 98 | 104 |
| Example 1-13 | Nonaqueous Electrolyte Solution 1-13 | (a-4) | 1.0 | - | - | 99 | 105 |
| Example 1-14 | Nonaqueous Electrolyte Solution 1-14 | (a-5) | 0.5 | - | - | 96 | 106 |
| Example 1-15 | Nonaqueous Electrolyte Solution 1-15 | (a-5) | 1.0 | - | - | 86 | 116 |
| Comparative Example 1-1 | Comparative Nonaqueous Electrolyte Solution 1-1 | - | - | - | - | 100 | 100 |
| Comparative Example 1-2 | Comparative Nonaqueous Electrolyte Solution 1-2 | (X) | 1.0 | - | - | 126 | 105 |
| Comparative Example 1-3 | Comparative Nonaqueous Electrolyte Solution 1-3 | (Y) | 1.0 | - | - | 130 | 107 |

**[Table 2]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 2-1 | Nonaqueous Electrolyte Solution 2-1 | (a-1) | 1.0 | VC | 1.0 | 80 | 105 |
| Example 2-2 | Nonaqueous Electrolyte Solution 2-2 | (a-2) | 1.0 | VC | 1.0 | 76 | 109 |
| Example 2-3 | Nonaqueous Electrolyte Solution 2-3 | (a-3) | 1.0 | VC | 1.0 | 81 | 108 |
| Comparative Example 2-1 | Comparative Nonaqueous Electrolyte Solution 2-1 | - | - | VC | 1.0 | 100 | 100 |
| Comparative Example 2-2 | Comparative Nonaqueous Electrolyte Solution 2-2 | (X) | 1.0 | VC | 1.0 | 122 | 102 |
| Comparative Example 2-3 | Comparative Nonaqueous Electrolyte Solution 2-3 | (Y) | 1.0 | VC | 1.0 | 130 | 104 |

**[Table 3]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 3-1 | Nonaqueous Electrolyte Solution 3-1 | (a-1) | 1.0 | BOB | 1.0 | 78 | 103 |
| Example 3-2 | Nonaqueous Electrolyte Solution 3-2 | (a-2) | 1.0 | BOB | 1.0 | 74 | 109 |
| Example 3-3 | Nonaqueous Electrolyte Solution 3-3 | (a-3) | 1.0 | BOB | 1.0 | 79 | 105 |
| Comparative Example 3-1 | Comparative Nonaqueous Electrolyte Solution 3-1 | - | - | BOB | 1.0 | 100 | 100 |
| Comparative Example 3-2 | Comparative Nonaqueous Electrolyte Solution 3-2 | (X) | 1.0 | BOB | 1.0 | 111 | 99 |
| Comparative Example 3-3 | Comparative Nonaqueous Electrolyte Solution 3-3 | (Y) | 1.0 | BOB | 1.0 | 117 | 102 |

**[Table 4]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 4-1 | Nonaqueous Electrolyte Solution 4-1 | (a-1) | 1.0 | DFBOP | 1.0 | 84 | 104 |
| Example 4-2 | Nonaqueous Electrolyte Solution 4-2 | (a-2) | 1.0 | DFBOP | 1.0 | 81 | 108 |
| Example 4-3 | Nonaqueous Electrolyte Solution 4-3 | (a-3) | 1.0 | DFBOP | 1.0 | 89 | 108 |
| Comparative Example 4-1 | Comparative Nonaqueous Electrolyte Solution 4-1 | - | - | DFBOP | 1.0 | 100 | 100 |
| Comparative Example 4-2 | Comparative Nonaqueous Electrolyte Solution 4-2 | (X) | 1.0 | DFBOP | 1.0 | 120 | 101 |
| Comparative Example 4-3 | Comparative Nonaqueous Electrolyte Solution 4-3 | (Y) | 1.0 | DFBOP | 1.0 | 136 | 103 |

**[Table 5]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 5-1 | Nonaqueous Electrolyte Solution 5-1 | (a-1) | 1.0 | TFOP | 1.0 | 89 | 105 |
| Example 5-2 | Nonaqueous Electrolyte Solution 5-2 | (a-2) | 1.0 | TFOP | 1.0 | 81 | 111 |
| Example 5-3 | Nonaqueous Electrolyte Solution 5-3 | (a-3) | 1.0 | TFOP | 1.0 | 84 | 107 |
| Comparative Example 5-1 | Comparative Nonaqueous Electrolyte Solution 5-1 | - | - | TFOP | 1.0 | 100 | 100 |
| Comparative Example 5-2 | Comparative Nonaqueous Electrolyte Solution 5-2 | (X) | 1.0 | TFOP | 1.0 | 121 | 102 |
| Comparative Example 5-3 | Comparative Nonaqueous Electrolyte Solution 5-3 | (Y) | 1.0 | TFOP | 1.0 | 127 | 104 |

**[Table 6]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 6-1 | Nonaqueous Electrolyte Solution 6-1 | (a-1) | 1.0 | FSI | 1.0 | 85 | 107 |
| Example 6-2 | Nonaqueous Electrolyte Solution 6-2 | (a-2) | 1.0 | FSI | 1.0 | 80 | 114 |
| Example 6-3 | Nonaqueous Electrolyte Solution 6-3 | (a-3) | 1.0 | FSI | 1.0 | 89 | 110 |
| Comparative Example 6-1 | Comparative Nonaqueous Electrolyte Solution 6-1 | - | - | FSI | 1.0 | 100 | 100 |
| Comparative Example 6-2 | Comparative Nonaqueous Electrolyte Solution 6-2 | (X) | 1.0 | FSI | 1.0 | 109 | 103 |
| Comparative Example 6-3 | Comparative Nonaqueous Electrolyte Solution 6-3 | (Y) | 1.0 | FSI | 1.0 | 113 | 105 |

**[Table 7]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 7-1 | Nonaqueous Electrolyte Solution 7-1 | (a-1) | 1.0 | DFP | 1.0 | 89 | 106 |
| Example 7-2 | Nonaqueous Electrolyte Solution 7-2 | (a-2) | 1.0 | DFP | 1.0 | 84 | 112 |
| Example 7-3 | Nonaqueous Electrolyte Solution 7-3 | (a-3) | 1.0 | DFP | 1.0 | 88 | 112 |
| Comparative Example 7-1 | Comparative Nonaqueous Electrolyte Solution 7-1 | - | - | DFP | 1.0 | 100 | 100 |
| Comparative Example 7-2 | Comparative Nonaqueous Electrolyte Solution 7-2 | (X) | 1.0 | DFP | 1.0 | 109 | 100 |
| Comparative Example 7-3 | Comparative Nonaqueous Electrolyte Solution 7-3 | (Y) | 1.0 | DFP | 1.0 | 138 | 105 |

**[Table 8]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 8-1 | Nonaqueous Electrolyte Solution 8-1 | (a-1) | 1.0 | FS | 1.0 | 86 | 108 |
| Example 8-2 | Nonaqueous Electrolyte Solution 8-2 | (a-2) | 1.0 | FS | 1.0 | 79 | 109 |
| Example 8-3 | Nonaqueous Electrolyte Solution 8-3 | (a-3) | 1.0 | FS | 1.0 | 84 | 108 |
| Comparative Example 8-1 | Comparative Nonaqueous Electrolyte Solution 8-1 | - | - | FS | 1.0 | 100 | 100 |
| Comparative Example 8-2 | Comparative Nonaqueous Electrolyte Solution 8-2 | (X) | 1.0 | FS | 1.0 | 118 | 101 |
| Comparative Example 8-3 | Comparative Nonaqueous Electrolyte Solution 8-3 | (Y) | 1.0 | FS | 1.0 | 123 | 105 |

**[Table 9]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 9-1 | Nonaqueous Electrolyte Solution 1-1 | (a-1) | 0.05 | - | - | 99 | 102 |
| Example 9-2 | Nonaqueous Electrolyte Solution 1-2 | (a-1) | 0.1 | - | - | 97 | 104 |
| Example 9-3 | Nonaqueous Electrolyte Solution 1-3 | (a-1) | 0.5 | - | - | 90 | 108 |
| Example 9-4 | Nonaqueous Electrolyte Solution 1-4 | (a-1) | 1.0 | - | - | 81 | 114 |
| Example 9-5 | Nonaqueous Electrolyte Solution 1-5 | (a-1) | 2.0 | - | - | 78 | 117 |
| Example 9-6 | Nonaqueous Electrolyte Solution 1-6 | (a-1) | 3.0 | - | - | 82 | 111 |
| Example 9-7 | Nonaqueous Electrolyte Solution 1-7 | (a-1) | 5.0 | - | - | 94 | 103 |
| Example 9-8 | Nonaqueous Electrolyte Solution 1-8 | (a-2) | 0.5 | - | - | 88 | 110 |
| Example 9-9 | Nonaqueous Electrolyte Solution 1-9 | (a-2) | 1.0 | - | - | 77 | 117 |
| Example 9-10 | Nonaqueous Electrolyte Solution 1-10 | (a-3) | 0.5 | - | - | 92 | 105 |
| Example 9-11 | Nonaqueous Electrolyte Solution 1-11 | (a-3) | 1.0 | - | - | 84 | 111 |
| Example 9-12 | Nonaqueous Electrolyte Solution 1-12 | (a-4) | 0.5 | - | - | 99 | 104 |
| Example 9-13 | Nonaqueous Electrolyte Solution 1-13 | (a-4) | 1.0 | - | - | 99 | 104 |
| Example 9-14 | Nonaqueous Electrolyte Solution 1-14 | (a-5) | 0.5 | | | 89 | 109 |
| Example 9-15 | Nonaqueous Electrolyte Solution 1-15 | (a-5) | 1.0 | | | 81 | 115 |
| Comparative Example 9-1 | Comparative Nonaqueous Electrolyte Solution 1-1 | - | - | - | - | 100 | 100 |
| Comparative Example 9-2 | Comparative Nonaqueous Electrolyte Solution 1-2 | (X) | 1.0 | - | - | 128 | 103 |
| Comparative Example 9-3 | Comparative Nonaqueous Electrolyte Solution 1-3 | (Y) | 1.0 | - | - | 126 | 106 |

**[Table 10]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 10-1 | Nonaqueous Electrolyte Solution 2-1 | (a-1) | 1.0 | VC | 1.0 | 84 | 105 |
| Example 10-2 | Nonaqueous Electrolyte Solution 2-2 | (a-2) | 1.0 | VC | 1.0 | 81 | 109 |
| Example 10-3 | Nonaqueous Electrolyte Solution 2-3 | (a-3) | 1.0 | VC | 1.0 | 82 | 106 |
| Comparative Example 10-1 | Comparative Nonaqueous Electrolyte Solution 2-1 | - | - | VC | 1.0 | 100 | 100 |
| Comparative Example 10-2 | Comparative Nonaqueous Electrolyte Solution 2-2 | (X) | 1.0 | VC | 1.0 | 114 | 101 |
| Comparative Example 10-3 | Comparative Nonaqueous Electrolyte Solution 2-3 | (Y) | 1.0 | VC | 1.0 | 127 | 101 |

**[Table 11]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 11-1 | Nonaqueous Electrolyte Solution 3-1 | (a-1) | 1.0 | BOB | 1.0 | 80 | 106 |
| Example 11-2 | Nonaqueous Electrolyte Solution 3-2 | (a-2) | 1.0 | BOB | 1.0 | 77 | 108 |
| Example 11-3 | Nonaqueous Electrolyte Solution 3-3 | (a-3) | 1.0 | BOB | 1.0 | 80 | 106 |
| Comparative Example 11-1 | Comparative Nonaqueous Electrolyte Solution 3-1 | - | - | BOB | 1.0 | 100 | 100 |
| Comparative Example 11-2 | Comparative Nonaqueous Electrolyte Solution 3-2 | (X) | 1.0 | BOB | 1.0 | 107 | 103 |
| Comparative Example 11-3 | Comparative Nonaqueous Electrolyte Solution 3-3 | (Y) | 1.0 | BOB | 1.0 | 115 | 102 |

**[Table 12]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 12-1 | Nonaqueous Electrolyte Solution 4-1 | (a-1) | 1.0 | DFBOP | 1.0 | 81 | 107 |
| Example 12-2 | Nonaqueous Electrolyte Solution 4-2 | (a-2) | 1.0 | DFBOP | 1.0 | 78 | 113 |
| Example 12-3 | Nonaqueous Electrolyte Solution 4-3 | (a-3) | 1.0 | DFBOP | 1.0 | 85 | 113 |
| Comparative Example 12-1 | Comparative Nonaqueous Electrolyte Solution 4-1 | - | - | DFBOP | 1.0 | 100 | 100 |
| Comparative Example 12-2 | Comparative Nonaqueous Electrolyte Solution 4-2 | (X) | 1.0 | DFBOP | 1.0 | 127 | 102 |
| Comparative Example 12-3 | Comparative Nonaqueous Electrolyte Solution 4-3 | (Y) | 1.0 | DFBOP | 1.0 | 132 | 105 |

**[Table 13]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 13-1 | Nonaqueous Electrolyte Solution 5-1 | (a-1) | 1.0 | TFOP | 1.0 | 88 | 106 |
| Example 13-2 | Nonaqueous Electrolyte Solution 5-2 | (a-2) | 1.0 | TFOP | 1.0 | 83 | 109 |
| Example 13-3 | Nonaqueous Electrolyte Solution 5-3 | (a-3) | 1.0 | TFOP | 1.0 | 84 | 107 |
| Comparative Example 13-1 | Comparative Nonaqueous Electrolyte Solution 5-1 | - | - | TFOP | 1.0 | 100 | 100 |
| Comparative Example 13-2 | Comparative Nonaqueous Electrolyte Solution 5-2 | (X) | 1.0 | TFOP | 1.0 | 118 | 101 |
| Comparative Example 13-3 | Comparative Nonaqueous Electrolyte Solution 5-3 | (Y) | 1.0 | TFOP | 1.0 | 124 | 103 |

**[Table 14]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 14-1 | Nonaqueous Electrolyte Solution 6-1 | (a-1) | 1.0 | FSI | 1.0 | 82 | 107 |
| Example 14-2 | Nonaqueous Electrolyte Solution 6-2 | (a-2) | 1.0 | FSI | 1.0 | 79 | 114 |
| Example 14-3 | Nonaqueous Electrolyte Solution 6-3 | (a-3) | 1.0 | FSI | 1.0 | 79 | 108 |
| Comparative Example 14-1 | Comparative Nonaqueous Electrolyte Solution 6-1 | - | - | FSI | 1.0 | 100 | 100 |
| Comparative Example 14-2 | Comparative Nonaqueous Electrolyte Solution 6-2 | (X) | 1.0 | FSI | 1.0 | 119 | 102 |
| Comparative Example 14-3 | Comparative Nonaqueous Electrolyte Solution 6-3 | (Y) | 1.0 | FSI | 1.0 | 130 | 103 |

**[Table 15]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 15-1 | Nonaqueous Electrolyte Solution 7-1 | (a-1) | 1.0 | DFP | 1.0 | 87 | 106 |
| Example 15-2 | Nonaqueous Electrolyte Solution 7-2 | (a-2) | 1.0 | DFP | 1.0 | 83 | 109 |
| Example 15-3 | Nonaqueous Electrolyte Solution 7-3 | (a-3) | 1.0 | DFP | 1.0 | 86 | 108 |
| Comparative Example 15-1 | Comparative Nonaqueous Electrolyte Solution 7-1 | - | - | DFP | 1.0 | 100 | 100 |
| Comparative Example 15-2 | Comparative Nonaqueous Electrolyte Solution 7-2 | (X) | 1.0 | DFP | 1.0 | 113 | 100 |
| Comparative Example 15-3 | Comparative Nonaqueous Electrolyte Solution 7-3 | (Y) | 1.0 | DFP | 1.0 | 125 | 101 |

**[Table 16]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 16-1 | Nonaqueous Electrolyte Solution 8-1 | (a-1) | 1.0 | FS | 1.0 | 80 | 108 |
| Example 16-2 | Nonaqueous Electrolyte Solution 8-2 | (a-2) | 1.0 | FS | 1.0 | 78 | 116 |
| Example 16-3 | Nonaqueous Electrolyte Solution 8-3 | (a-3) | 1.0 | FS | 1.0 | 79 | 108 |
| Comparative Example 16-1 | Comparative Nonaqueous Electrolyte Solution 8-1 | - | - | FS | 1.0 | 100 | 100 |
| Comparative Example 16-2 | Comparative Nonaqueous Electrolyte Solution 8-2 | (X) | 1.0 | FS | 1.0 | 121 | 103 |
| Comparative Example 16-3 | Comparative Nonaqueous Electrolyte Solution 8-3 | (Y) | 1.0 | FS | 1.0 | 137 | 104 |

**[Table 17]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 17-1 | Nonaqueous Electrolyte Solution 9-1 | (a-1 (Na)) | 0.5 | - | - | 83 | 107 |
| Example 17-2 | Nonaqueous Electrolyte Solution 9-2 | (a-1 (Na)) | 1.0 | - | - | 80 | 115 |
| Example 17-3 | Nonaqueous Electrolyte Solution 9-3 | (a-2 (Na)) | 0.5 | - | - | 79 | 108 |
| Example 17-4 | Nonaqueous Electrolyte Solution 9-4 | (a-2 (Na)) | 1.0 | - | - | 77 | 113 |
| Example 17-5 | Nonaqueous Electrolyte Solution 9-5 | (a-3 (Na)) | 0.5 | - | - | 78 | 107 |
| Example 17-6 | Nonaqueous Electrolyte Solution 9-6 | (a-3 (Na)) | 1.0 | - | - | 77 | 115 |
| Example 17-7 | Nonaqueous Electrolyte Solution 9-7 | (a-4 (Na)) | 0.5 | | | 94 | 103 |
| Example 17-8 | Nonaqueous Electrolyte Solution 9-8 | (a-4 (Na)) | 1.0 | - | - | 98 | 106 |
| Example 17-9 | Nonaqueous Electrolyte Solution 9-9 | (a-5 (Na)) | 0.5 | - | - | 84 | 106 |
| Example 17-10 | Nonaqueous Electrolyte Solution 9-10 | (a-5 (Na)) | 1.0 | - | - | 79 | 116 |
| Comparative Example 17-1 | Comparative Nonaqueous Electrolyte Solution 9-1 | - | - | - | - | 100 | 100 |
| Comparative Example 17-2 | Comparative Nonaqueous Electrolyte Solution 9-2 | (X) | 1.0 | - | - | 105 | 102 |

**[Table 18]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 18-1 | Nonaqueous Electrolyte Solution 10-1 | (a-1 (Na)) | 0.5 | VC | 0.5 | 86 | 106 |
| Example 18-2 | Nonaqueous Electrolyte Solution 10-2 | (a-2 (Na)) | 0.5 | VC | 0.5 | 81 | 108 |
| Example 18-3 | Nonaqueous Electrolyte Solution 10-3 | (a-3 (Na)) | 0.5 | VC | 0.5 | 82 | 108 |
| Comparative Example 18-1 | Comparative Nonaqueous Electrolyte Solution 10-1 | - | - | VC | 0.5 | 100 | 100 |
| Comparative Example 18-2 | Comparative Nonaqueous Electrolyte Solution 10-2 | (X) | 0.5 | VC | 0.5 | 107 | 101 |

**[Table 19]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 19-1 | Nonaqueous Electrolyte Solution 11-1 | (a-1 (Na)) | 0.5 | NaSO₃F | 1.0 | 91 | 104 |
| Example 19-2 | Nonaqueous Electrolyte Solution 11-2 | (a-2 (Na)) | 0.5 | NaSO₃F | 1.0 | 85 | 107 |
| Example 19-3 | Nonaqueous Electrolyte Solution 11-3 | (a-3 (Na)) | 0.5 | NaSO₃F | 1.0 | 88 | 106 |
| Comparative Example 19-1 | Comparative Nonaqueous Electrolyte Solution 11-1 | - | - | NaSO₃F | 1.0 | 100 | 100 |
| Comparative Example 19-2 | Comparative Nonaqueous Electrolyte Solution 11-2 | (X) | 0.5 | NaSO₃F | 1.0 | 112 | 99 |

**[Table 20]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 20-1 | Nonaqueous Electrolyte Solution 12-1 | (a-1 (Na)) | 0.5 | TFOP-Na | 1.0 | 82 | 106 |
| Example 20-2 | Nonaqueous Electrolyte Solution 12-2 | (a-2 (Na)) | 0.5 | TFOP-Na | 1.0 | 79 | 107 |
| Example 20-3 | Nonaqueous Electrolyte Solution 12-3 | (a-3 (Na)) | 0.5 | TFOP-Na | 1.0 | 83 | 107 |
| Comparative Example 20-1 | Comparative Nonaqueous Electrolyte Solution 12-1 | - | - | TFOP-Na | 1.0 | 100 | 100 |
| Comparative Example 20-2 | Comparative Nonaqueous Electrolyte Solution 12-2 | (X) | 0.5 | TFOP-Na | 1.0 | 106 | 102 |

**[Table 21]**

| | Nonaqueous electrolyte solution | Component (I) or comparative compound | | Another additive | | Resistance after high-temperature cycle test (relative value) | Discharge capacity retention rate after high-temperature cycle (relative value) |
|---|---|---|---|---|---|---|---|
| | | Type | Content [% by mass] | Type | Content [% by mass] | | |
| Example 21-1 | Nonaqueous Electrolyte Solution 13-1 | (a-1 (Na)) | 0.5 | DFP-Na | 0.5 | 88 | 108 |
| Example 21-2 | Nonaqueous Electrolyte Solution 13-2 | (a-2 (Na)) | 0.5 | DFP-Na | 0.5 | 83 | 110 |
| Example 21-3 | Nonaqueous Electrolyte Solution 13-3 | (a-3 (Na)) | 0.5 | DFP-Na | 0.5 | 84 | 107 |
| Comparative Example 21-1 | Comparative Nonaqueous Electrolyte Solution 13-1 | - | - | DFP-Na | 0.5 | 100 | 100 |
| Comparative Example 21-2 | Comparative Nonaqueous Electrolyte Solution 13-2 | (X) | 0.5 | DFP-Na | 0.5 | 113 | 103 |

As is clear from Tables 1 to 21, it was found that a nonaqueous electrolyte solution battery using a nonaqueous electrolyte solution including Component (I) of the present disclosure had excellent high-temperature cycle characteristics and could suppress an increase in battery resistance.

### INDUSTRIAL APPLICABILITY

The present disclosure allows for providing a nonaqueous electrolyte solution and a nonaqueous electrolyte solution battery that can improve high-temperature cycle characteristics and suppress an increase in battery resistance. In addition, the present disclosure also allows for providing a compound and an additive for nonaqueous electrolyte solution that can be suitably used in the above nonaqueous electrolyte solution.

Although the present disclosure has been described in detail and with reference to specific examples, it will be apparent to those skilled in the art that various changes and modifications can be made without departing from the spirit and scope of the present disclosure.

The present application is based on a Japanese Patent Application No. 2021-162010 filed on September 30, 2021, the contents of which are incorporated herein by reference.

## Claims

1. A nonaqueous electrolyte solution, comprising:
(I) a compound represented by the following Formula (1);
(II) a solute; and
(III) a nonaqueous organic solvent, 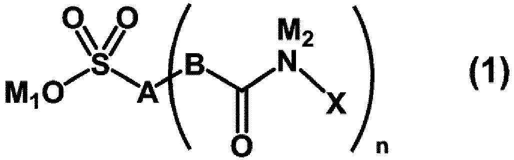
where, A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms, at least one hydrogen atom in the organic group may be substituted with a fluorine atom, and an oxygen atom may be present in the organic group;
B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, and at least one hydrogen atom in the organic group may be substituted with a halogen atom;
M₁ and M₂ each independently represent a hydrogen atom, a metal cation, or an onium cation, and when M₁ and M₂ represent a metal cation or an onium cation, a bond between the oxygen atom and M₁ and a bond between a nitrogen atom and M₂ in Formula (1) represent an ionic bond;
X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group may be substituted with a fluorine atom, at least one of oxygen atom or unsaturated bond may be present in the organic group, and R_{c} and R_{d} may bond to each other to form a cyclic structure; and
n represents an integer of 1 to 4.

2. The nonaqueous electrolyte solution according to claim 1,
wherein X in Formula (1) represents -S(=O)₂-R_{b}, and the R_{b} represents a fluorine atom.

3. The nonaqueous electrolyte solution according to claim 1,
wherein A in Formula (1) represents a linear alkylene group having 1 to 4 carbon atoms or a branched alkylene group having 2 to 4 carbon atoms.

4. The nonaqueous electrolyte solution according to claim 1,
wherein the solute is at least one selected from the group consisting of LiPF₆, LiBF₄, LiSbF₆, LiAsF₆, LiClO₄, LiCF₃SO₃, LiC₄F₉SO₃, LiN(SO₂F)₂, LiAlO₂, LiAlCl₄, LiCl, and LiI, or at least one selected from the group consisting of NaPF₆, NaBF₄, NaSbF₆, NaAsF₆, NaClO₄, NaCF₃SO₃, NaC₄F₉SO₃, NaN(SO₂F)₂, NaAlO₂, NaAlCl₄, NaCl, and NaI.

5. The nonaqueous electrolyte solution according to claim 1,
wherein the nonaqueous organic solvent is at least one selected from the group consisting of a cyclic ester, a chain ester, a cyclic ether, a chain ether, a sulfone compound, a sulfoxide compound, and an ionic liquid.

6. The nonaqueous electrolyte solution according to claim 5,
wherein the nonaqueous organic solvent contains a cyclic ester, and the cyclic ester is cyclic carbonate.

7. The nonaqueous electrolyte solution according to claim 6,
wherein the cyclic carbonate is at least one selected from the group consisting of ethylene carbonate, propylene carbonate, and fluoroethylene carbonate.

8. The nonaqueous electrolyte solution according to claim 5,
wherein the nonaqueous organic solvent contains a chain ester, and the chain ester is chain carbonate.

9. The nonaqueous electrolyte solution according to claim 8,
wherein the chain carbonate is at least one selected from the group consisting of ethyl methyl carbonate, dimethyl carbonate, diethyl carbonate, and methyl propyl carbonate.

10. The nonaqueous electrolyte solution according to claim 1,
wherein a content of the (I) is 0.01% by mass to 5.0% by mass with respect to a total amount of the nonaqueous electrolyte solution.

11. The nonaqueous electrolyte solution according to claim 1, further comprising:
at least one selected from vinylene carbonate, bis(oxalato)borate, difluorooxalatoborate, difluorobis(oxalato)phosphate, tetrafluorooxalatophosphate, (difluorophosphoryl)(fluorosulfonyl)imide salt, difluorophosphate, fluorosulfonate, 1,3-propenesultone, 1,3-propanesultone, 1,6-diisocyanatohexane, ethynylethylene carbonate, 1,3,2-dioxathiolane-2,2-dioxide, 4-propyl-1,3,2-dioxathiolane-2,2-dioxide, methylene methanedisulfonate, 1,2-ethanedisulfonic anhydride, methanesulfonyl fluoride, tris(trimethylsilyl)borate, (ethoxy)pentafluorocyclotriphosphazene, tetrafluoro(malonato)phosphate, tetrafluoro(picolinato)phosphate, 1,3-dimethyl-1,3-divinyl-1,3-di(1,1,1,3,3,3-hexafluoroisopropyl)disiloxane, tetravinylsilane, t-butylbenzene, t-amylbenzene, fluorobenzene, and cyclohexylbenzene.

12. A nonaqueous electrolyte solution battery, at least comprising:
a positive electrode;
a negative electrode;
a separator; and
the nonaqueous electrolyte solution according to any one of claims 1 to 11.

13. A compound represented by the following Formula (1), 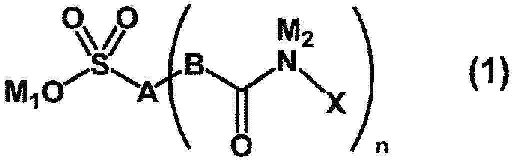 where, A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms, at least one hydrogen atom in the organic group may be substituted with a fluorine atom, and an oxygen atom may be present in the organic group;
B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, and at least one hydrogen atom in the organic group may be substituted with a halogen atom;
M₁ and M₂ each independently represent a hydrogen atom, a metal cation, or an onium cation, and when M₁ and M₂ represent a metal cation or an onium cation, a bond between the oxygen atom and M₁ and a bond between a nitrogen atom and M₂ in Formula (1) represent an ionic bond;
X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group may be substituted with a fluorine atom, at least one of oxygen atom or unsaturated bond may be present in the organic group, and R_{c} and R_{d} may bond to each other to form a cyclic structure; and
n represents an integer of 1 to 4.

14. The compound according to claim 13,
wherein X in Formula (1) represents -S(=O)₂-R_{b}, and the R_{b} represents a fluorine atom.

15. The compound according to claim 13 or 14,
wherein A in Formula (1) represents a linear alkylene group having 1 to 4 carbon atoms or a branched alkylene group having 2 to 4 carbon atoms.

16. An additive for nonaqueous electrolyte solution represented by the following Formula (1), 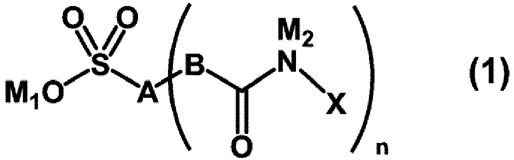 where, A represents an organic group selected from the group consisting of a linear alkylene group having 1 to 8 carbon atoms or a branched alkylene group having 2 to 8 carbon atoms, and a linear alkenylene group having 2 to 8 carbon atoms or a branched alkenylene group having 3 to 8 carbon atoms, at least one hydrogen atom in the organic group may be substituted with a fluorine atom, and an oxygen atom may be present in the organic group;
B represents an oxygen atom or NRₐ, and Rₐ represents a hydrogen atom or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, and an aryl group having 6 to 10 carbon atoms, and at least one hydrogen atom in the organic group may be substituted with a halogen atom;
M₁ and M₂ each independently represent a hydrogen atom, a metal cation, or an onium cation, and when M₁ and M₂ represent a metal cation or an onium cation, a bond between the oxygen atom and M₁ and a bond between a nitrogen atom and M₂ in Formula (1) represent an ionic bond;
X represents -S(=O)₂-R_{b} or -P(=O)-R_{c}R_{d}, R_{b} to R_{d} each independently represent a fluorine atom, or an organic group selected from the group consisting of an alkyl group having 1 to 10 carbon atoms, an alkenyl group having 2 to 10 carbon atoms, an alkynyl group having 2 to 10 carbon atoms, a cycloalkyl group having 3 to 10 carbon atoms, a cycloalkenyl group having 3 to 10 carbon atoms, an aryl group having 6 to 10 carbon atoms, an alkoxy group having 1 to 10 carbon atoms, an alkenyloxy group having 2 to 10 carbon atoms, an alkynyloxy group having 2 to 10 carbon atoms, a cycloalkoxy group having 3 to 10 carbon atoms, a cycloalkenyloxy group having 3 to 10 carbon atoms, and an aryloxy group having 6 to 10 carbon atoms, at least one hydrogen atom in the organic group may be substituted with a fluorine atom, at least one of oxygen atom or unsaturated bond may be present in the organic group, and R_{c} and R_{d} may bond to each other to form a cyclic structure; and
n represents an integer of 1 to 4.

17. The additive for nonaqueous electrolyte solution according to claim 16,
wherein X in Formula (1) represents -S(=O)₂-R_{b}, and the R_{b} represents a fluorine atom.

18. The additive for nonaqueous electrolyte solution according to claim 16 or 17,
wherein A in Formula (1) represents a linear alkylene group having 1 to 4 carbon atoms or a branched alkylene group having 2 to 4 carbon atoms.
